# EUROPEAN PATENT APPLICATION

(11) **EP 3 603 639 A1**
(43) Date of publication of application: **05.02.2020**
(21) Application number: 18775246.4
(22) Date of filing: 30.03.2018
(51) Int. Cl.: A61K 31/454, A61K 31/4178, A61K 31/496, A61P 25/02, C07D 401/06, C07D 403/06

(54) **THERAPEUTIC OR PROPHYLACTIC AGENT FOR PERIPHERAL NEUROPATHIES**

(30) Priority: 31.03.2017 JP 2017071339; 31.03.2017 JP 2017071329
(71) Applicant: Toray Industries, Inc., Tokyo 103-8666 (JP)
(72) Inventor: HARA, Kojiro, Kamakura-shi Kanagawa 248-8555 (JP); SUZUKI, Tomohiko, Kamakura-shi Kanagawa 248-8555 (JP); YOSHIDA, Chihiro, Kamakura-shi Kanagawa 248-8555 (JP); TAKEO, Koji, Kamakura-shi Kanagawa 248-8555 (JP); SHIMODA, Koji, Kamakura-shi Kanagawa 248-8555 (JP); IZUMIMOTO, Naoki, Kamakura-shi Kanagawa 248-8555 (JP); NISHIMURA, Kazumi, Kamakura-shi Kanagawa 248-8555 (JP); NAGURO, Rieko, Kamakura-shi Kanagawa 248-8555 (JP)
(74) Representative: Kador & Partner PartG mbB
(86) International application number: PCT/JP2018/013536
(87) International publication number: WO 2018/181860

(57) **Abstract**

The present invention addresses the problem of providing a compound for treatment or prevention of peripheral neuropathies. Provided is a therapeutic or prophylactic agent for peripheral neuropathies, the agent containing, as an active ingredient, a cyclic amine derivative represented by the chemical formula indicated herein or a pharmacologically acceptable salt thereof

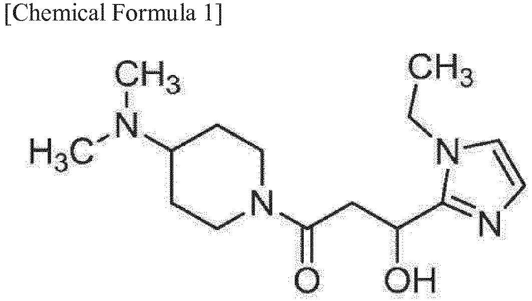

## Description

### TECHNICAL FIELD

The present invention relates to a therapeutic or prophylactic agent for peripheral neuropathies.

### BACKGROUND ART

Peripheral neuropathy is induced by injury to neurons (axons or cell bodies) or myelin sheaths (Schwann cells) constituting peripheral nerves. Histopathologically, axonal degeneration and myelin sheath degeneration are observed, and physiologically, dysfunctions such as a decrease in nerve conduction velocity, take place.

It is considered that injury to neurons or myelin sheaths of peripheral nerves induces peripheral neuropathies such as sensory neuropathies exhibiting symptoms such as numbness of limbs (dysesthesia), paresthesia, hypesthesia, pain, hypacusia, or the like, motor neuropathies exhibiting symptoms such as muscle weakness or atrophy, flaccid paralysis or deep tendon reflex decrease or loss, or the like, or, autonomic neuropathies exhibiting symptoms such as constipation, abdominal pain, dyshidrosis, dysuria, orthostatic hypotension, or the like (Non Patent Literature 1).

These symptoms of peripheral neuropathies are not life-threatening in most cases but have a large impact on the patients' daily life and significantly lower their quality of life (Non Patent Literature 1).

Peripheral neuropathies can be roughly classified depending on the causes of damaging nerve. Typical examples thereof include drug-induced peripheral neuropathies, autoimmune peripheral neuropathies, metabolic peripheral neuropathies, and hereditary peripheral neuropathies.

Examples of drugs inducing drug-induced peripheral neuropathies include anticancer agents, antiviral agents, antimicrobial agents, antitubercular agents, antiarrhythmic agents, lipid-lowering drugs, immunosuppressive drugs, and gout therapeutic agents. The symptoms of drug-induced peripheral neuropathies are often mainly composed of sensory disturbances such as pain, and such disturbances can remain after drug withdrawal (Non Patent Literature 2).

In particular, anticancer agents are also problematic in that these agents cause peripheral neuropathies at high incidence rates, making it difficult to continue the cancer therapy. In order to relieve the symptoms of peripheral neuropathies induced by anticancer agents, analgesics (for example, pregabalin, gabapentin or ketamine), antiepileptic agents (for example, lamotrigine, carbamazepine, phenytoin, valproic acid or clonazepam), antidepressants (for example, amitriptyline, imipramine, clomipramine or duloxetine), Chinese herbal medicines (for example, Goshajinkigan extract or Shakuyakukanzoto extract), vitamin B formulations (for example, B6 or B12) or the like are administered. However, no method for effectively treating or preventing peripheral neuropathies induced by anticancer agents has been established (Non Patent Literature 1).

Among the above drugs, duloxetine alone exhibits high evidence levels in clinical trials, and the use thereof is recommended in the guideline for treating chemotherapy-induced peripheral neuropathy, which has been developed by the American Society of Clinical Oncology (Non Patent Literature 3). Meanwhile, among drugs, the uses of which are recommended in the guidelines for treating neuropathic pain, developed by the International Association for the Study of Pain and the European nerve society, respectively (Non Patent Literature 4 and 5), no evidence exists, which clearly supports the fact that all of pregabalin, gabapentin, nortriptyline, and amitriptyline, excluding duloxetine, are effective for neuropathic pain induced by anticancer agents (Non Patent Literature 6 and 7).

Autoimmune peripheral neuropathies are neuropathies induced by autoimmunity against the components of peripheral nerves, and are various disease groups including Guillain-Barré syndrome (GBS), chronic inflammatory demyelinating polyradiculoneuropathy (CIDP), multifocal motor neuropathy (MMN), and paraproteinemic neuropathy (PPN) (Non Patent Literature 8).

It is considered that GBS is induced by infection with pathogenic microorganisms such as viruses and bacteria as a trigger, and GBS may be developed after vaccination for preventing infection with a pathogenic microorganism. GBS has cardinal signs including quadriplegia and absent deep reflex, and is often accompanied by sensory disturbances such as pain and dysesthesia. When GBS is severe, patients can die of respiratory disorder or autonomic neuropathy. GBS has many subtypes and acute inflammatory demyelinating polyneuropathy, acute motor axonal neuropathy, acute motor-sensory axonal neuropathy, Fisher syndrome, and the like are known (Non Patent Literature 9). CIDP differs from GBS and is a chronic or relapsing or ameliorative autoimmune peripheral neuropathy leading to muscle weakness and sensory disturbances. MMN and PPN are diseases analogous to CIDP. MMN is characterized by myopathy not accompanied by sensory disturbances (Non Patent Literature 10). PPN is induced by abnormal proliferation of a homogeneous immunoglobulin, and is characterized by slowly progressive sensory neuropathy (Non Patent Literature 11).

As methods for treating autoimmune peripheral neuropathies, an intravenous immunoglobulin therapy (IVIg therapy) and simple plasma exchange therapy are said to be effective (Non Patent Literature 12). However, the simple plasma exchange therapy has disadvantages such that it requires special facilities and equipment, and cannot be applied for elderly people or patients with circulatory insufficiency, for example. Meanwhile, the intravenous immunoglobulin therapy (IVIg therapy) requires the judicious use thereof for patients with past histories including shock and hypersensitivity. As described above, a therapeutic drug that can be conveniently used and has few side effects has been desired in medical practice.

Metabolic peripheral neuropathies are caused by various metabolic abnormalities. Diseases causing metabolic peripheral neuropathies vary widely, such as diabetes, uremia, collagen disease, avitaminosis, and hypothyroidism.

In particular, diabetes is the most frequent cause of peripheral neuropathies, and the number of diabetic patients is predicted to increase in the future. One of mechanisms of causing the onset of diabetic peripheral neuropathy is hyperactivity of the polyol pathway that metabolizes glucose into sorbitol. It is considered that excessively accumulated sorbitol injures neurons (Non Patent Literature 13). Accordingly, inhibitors for aldose reductase involving the polyol pathway are considered as effective against diabetic peripheral neuropathy, however, epalrestat alone has been approved in Japan, it exerts its effects only among patients with relatively mild pathological conditions, and it is often ineffective for severely affected patients or patients with long duration of disease (Non Patent Literature 14). Furthermore, pregabalin, duloxetine and the like are used for pain due to diabetic peripheral neuropathies. However, they are not drugs against peripheral neuropathies, so that a new drug exhibiting significant effects on diabetic peripheral neuropathy is desired.

Examples of hereditary peripheral neuropathies include Charcot-Marie-Tooth disease, familial amyloid polyneuropathy, hereditary neuropathy to pressure palsies (HNPP), and hereditary neuralgic amyotrophy. The most typical example thereof is Charcot-Marie-Tooth disease. At least 50 types of genes considered to be involved in Charcot-Marie-Tooth disease are known, and characterized in that they are mutated genes involving in myelination, formation or maintenance of neurons, and the like, and various heterogenes exist. Generally, in most cases of hereditary peripheral neuropathies, motor nerves and sensory nerves are damaged and motor difficulty is significant. Physical therapy or occupational therapy may be performed in order to clinically maintain muscle strength. However, there is currently no therapeutic method and/or drug effective against hereditary peripheral neuropathies including Charcot-Marie-Tooth disease (Non Patent Literature 15).

In the case of peripheral neuropathies induced by various causes, there are patients for whom no effective drug exists or patients for whom drugs exist but are ineffective. Hence, creation of a new drug against peripheral neuropathies is expected.

Moreover, in clinical practice, differential diagnosis of the causes of peripheral neuropathies from one another needs detailed examination, and some cases are diagnosed as idiopathic peripheral neuropathies (Non Patent Literature 16), for example. Hence, a drug, which is effective against overall peripheral neuropathies independently from the causes of neuropathies, is extremely useful, but no such drug exists currently. However, even so, it is possible to create a drug, which is effective against overall peripheral neuropathies. This is because, as described above, peripheral neuropathies are divided into many types, and the clinical symptoms thereof are also varied, but these neuropathies share a feature such that the neuropathies are developed by injury to cells constituting peripheral nerves, in a manner independent from the causes of the onset. Therefore, a drug on the basis of a neurotrophic factor that is an *in vivo* molecule involving the survival, growth or maintenance of neurons, for example, is predicted to be broadly effective against peripheral neuropathies (Non Patent Literature 17). However, even if a drug is created on the basis of a neurotrophic factor, the efficacy of such a drug has not been confirmed in clinical trials on anticancer agent-induced peripheral neuropathies or diabetic peripheral neuropathies (Non Patent Literature 18 and 19), demonstrating that creation of a drug effective against overall peripheral neuropathies is extremely difficult.

Patent Literature 1 discloses that a cyclic amine derivative has analgesic action, but discloses no report suggesting its effect on peripheral neuropathies.

### CITATION LIST

### PATENT LITERATURE

Patent Literature 1: WO2016/136944

### NON PATENT LITERATURE

Non Patent Literature 1: Shizuoka Cancer Center, "Treatment with Anticancer Agent and Peripheral Neuropathy (3rd printing)", 2016, p. 1-36
Non Patent Literature 2: Vilholm et al., Basic & Clinical Pharmacology & Toxicology, 2014, Vol. 115, p.185-192
Non Patent Literature 3: Hershman et al. Journal of Oncology Practice, 2014, Vol. 10, p. e421-e424
Non Patent Literature 4: Attal et al. Pain: Clinical Updates, 2010, Vol. 18
Non Patent Literature 5: Attal et al. European Journal of Neurology, 2010, Vol. 17, p.1113-1123
Non Patent Literature 6: Shinde et al. Support Care Cancer, 2016, Vol. 24, p. 547-553
Non Patent Literature 7: Gewandter et al. Pain, 2017, Vol. 158, p. 30-33
Non Patent Literature 8: Kusunoki, Clinical Neurology, 2009, Vol. 49, p. 956-958
Non Patent Literature 9: Hughes et al. The Lancet, 2005, Vol. 366, p. 1653-1666
Non Patent Literature 10: Kusunoki, Internal Medicine, The Japanese Society of Internal Medicine, 2013, Vol. 102, p.1965-1970
Non Patent Literature 11: Rison et al. BioMed Central Neurology, 2016, Vol. 16, No. 13
Non Patent Literature 12: Hughes et al. The Lancet, 1997, Vol. 349, p. 225-230
Non Patent Literature 13: Singh et al. Pharmacological Research, 2014, Vol. 80, p. 21-35
Non Patent Literature 14: Schemmel et al. Journal of Diabetes and Its Complication, 2010, Vol. 24, p. 354-360
Non Patent Literature 15: Saporta et al. Neurologic Clinics, 2013, Vol. 31, p. 597-619
Non Patent Literature 16: Azhary et al. American Family Physician, 2010, Vol. 81, p. 887-892
Non Patent Literature 17: McMahon et al. Current Opinion in Neurobiology, 1995, Vol. 5, p. 616-624
Non Patent Literature 18: Argyriou et al. Critical Reviews in Oncology/Hematology, 2012, Vol. 82, p. 51-77
Non Patent Literature 19: Apfel et al. JAMA, 2000, Vol. 284, p. 2215-2221

### SUMMARY OF INVENTION

### TECHNICAL PROBLEM

An object of the present invention is to provide a therapeutic or prophylactic agent for peripheral neuropathies.

### SOLUTION TO PROBLEM

As a result of intensive studies to achieve the above object, the present inventors have discovered that a specific cyclic amine derivative or a pharmacologically acceptable salt thereof has a significant effect of suppressing peripheral neuropathies.

Specifically, the present invention provides a therapeutic agent or a prophylactic agent for peripheral neuropathies, comprising as an active ingredient, a cyclic amine derivative represented by the following general formula (I) or a pharmacologically acceptable salt thereof. 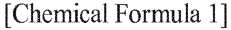 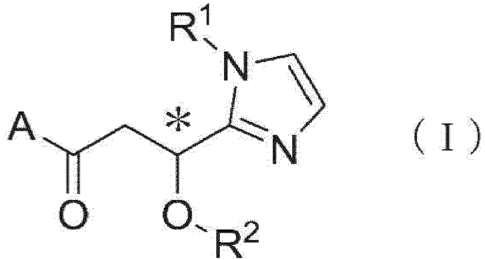 [wherein, carbon marked with * is asymmetric carbon, and A represents a group represented by general formulae (IIa), (IIb) or (IIc): 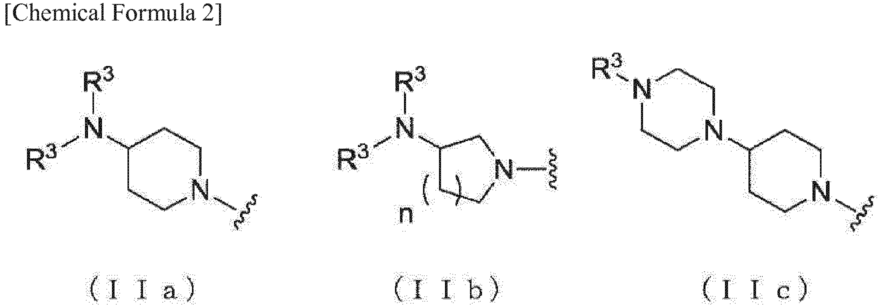 wherein R¹ represents a methyl group or an ethyl group optionally substituted with a halogen atom, R² represents a hydrogen atom or an alkylcarbonyl group having 2 to 5 carbon atoms, each R³ independently represents a methyl group or an ethyl group, and n represents 1 or 2.]

In the aforementioned cyclic amine derivative, it is preferable that A is the group represented by general formula (IIa), in which R¹ is more preferably a methyl group or an ethyl group optionally substituted with fluorine atom; and further preferably a methyl group, an ethyl group, a difluoromethyl group or a 2,2,2-trifluoroethyl group.

In the above cyclic amine derivative, it is preferable that A is the group represented by general formulae (IIb) or (IIc), in which R¹ is more preferably a methyl group or an ethyl group optionally substituted with a fluorine atom, and further preferably a methyl group, an ethyl group, a difluoromethyl group or a 2,2,2-trifluoroethyl group.

In the above cyclic amine derivative, it is preferable that A is the group represented by general formula (IIa) and that the stereochemical configuration of the asymmetric carbon marked with * is preferably S, in which R¹ is more preferably a methyl group or an ethyl group optionally substituted with a fluorine atom, and further preferably a methyl group, an ethyl group, a difluoromethyl group or a 2,2,2-trifluoroethyl group.

The present invention also provides a pharmaceutical composition for treating or preventing peripheral neuropathies, containing a cyclic amine derivative represented by general formula (I) or a pharmacologically acceptable salt thereof, and a pharmacologically acceptable excipient, and the like.

The present invention also provides a cyclic amine derivative represented by general formula (I) or a pharmacologically acceptable salt thereof for use in treatment or prevention of peripheral neuropathies.

The present invention also provides use of a cyclic amine derivative represented by general formula (I) or a pharmacologically acceptable salt thereof in treatment or prevention of peripheral neuropathies.

The present invention also provides use of a cyclic amine derivative represented by general formula (I) or a pharmacologically acceptable salt thereof in producing a medicine for treatment or prevention of peripheral neuropathies.

The present invention also provides a method for treating or preventing peripheral neuropathies, which includes administering a therapeutically effective amount of a cyclic amine derivative represented by general formula (I) or a pharmacologically acceptable salt thereof to a patient who needs treatment.

In each embodiment of the present invention, the above peripheral neuropathies are preferably drug-induced peripheral neuropathies, autoimmune peripheral neuropathies, metabolic peripheral neuropathies, hereditary peripheral neuropathies, vasculitic peripheral neuropathies, toxic peripheral neuropathies, infectious peripheral neuropathies, or peripheral neuropathies associated with malignant tumor, more preferably drug-induced peripheral neuropathies, autoimmune peripheral neuropathies, metabolic peripheral neuropathies, or hereditary peripheral neuropathies, and further preferably drug-induced peripheral neuropathies, autoimmune peripheral neuropathies, or metabolic peripheral neuropathies. The above drug-induced peripheral neuropathies are preferably anticancer agent-induced peripheral neuropathies. The above autoimmune peripheral neuropathies are preferably at least one type selected from Guillain-Barré syndrome (GBS), chronic inflammatory demyelinating polyradiculoneuropathy (CIDP), multifocal motor neuropathy (MMN), and paraproteinemic neuropathy (PPN). The above metabolic peripheral neuropathies are preferably diabetic peripheral neuropathy. The above hereditary peripheral neuropathy is preferably Charcot-Marie-Tooth disease.

### ADVANTAGEOUS EFFECTS OF INVENTION

With the cyclic amine derivative or the pharmacologically acceptable salt thereof of the present invention, peripheral neuropathies can be treated or prevented. The above peripheral neuropathies are for example, drug-induced peripheral neuropathies, autoimmune peripheral neuropathies, or metabolic peripheral neuropathies. The above drug-induced peripheral neuropathies are particularly anticancer agent-induced peripheral neuropathies. The above autoimmune peripheral neuropathies are particularly at least one type selected from Guillain-Barré syndrome (GBS), chronic inflammatory demyelinating polyradiculoneuropathy (CIDP), multifocal motor neuropathy (MMN), and paraproteinemic neuropathy (PPN). The above metabolic peripheral neuropathies are particularly diabetic peripheral neuropathies. The above hereditary peripheral neuropathy is particularly Charcot-Marie-Tooth disease.

This description includes the contents as disclosed in the specifications and/or drawings of Japanese Patent Application Nos. 2017-071329 and 2017-071339, which are priority literatures of the present application.

### BRIEF DESCRIPTION OF DRAWINGS

[Figure 1] Figure 1 shows the protective effect of compound 1 on cell damage in a rat dorsal root ganglion-derived established neuronal cell line.
[Figure 2] Figure 2 shows repairing effect of compound 1 on cell damage in a rat dorsal root ganglion-derived established neuronal cell line.
[Figure 3] Figure 3 shows the effect of compound 1 on myelination in the co-culture of rat dorsal root ganglion neurons and Schwann cells.
[Figure 4] Figure 4 shows the effect of compound 1 on the expression level of myelin basic protein in the co-culture of rat dorsal root ganglion neurons and Schwann cells.
[Figure 5] Figure 5 shows the effect of the repeated dosing of compound 1 on cold allodynia in a rat oxaliplatin-induced peripheral neuropathy model.
[Figure 6] Figure 6 shows the effect of the repeated dosing of compound 1 on mechanical allodynia in a rat oxaliplatin-induced peripheral neuropathy model.
[Figure 7] Figure 7 shows the effect of the single dosing of compound 1 on mechanical allodynia in a rat cisplatin-induced peripheral neuropathy model.
[Figure 8] Figure 8 shows the effect of the single dosing of compound 1 on mechanical allodynia in a rat paclitaxel-induced peripheral neuropathy model.
[Figure 9] Figure 9 shows the effect of the single dosing of compound 1 on mechanical allodynia in a rat bortezomib-induced peripheral neuropathy model.
[Figure 10] Figure 10 shows the effect of compound 1 on the clinical scores of a rat experimental autoimmune neuritis model.
[Figure 11] Figure 11 shows the effect of compound 1 on body weight loss in a rat experimental autoimmune neuritis model.
[Figure 12] Figure 12 shows the effect of compound 1 on mechanical allodynia in a rat experimental autoimmune neuritis model.
[Figure 13] Figure 13 shows the effect of compound 1 on decreased nerve conduction velocity in a rat streptozotocin-induced diabetes model.
[Figure 14] Figure 14 shows the effect of compound 1 on mechanical allodynia in a rat streptozotocin-induced diabetes model.

### DESCRIPTION OF EMBODIMENTS

The following terms used in the specification are, unless otherwise specified, defined as follows.

It is characterized in that the cyclic amine derivative according to an embodiment of the present invention is represented by the following general formula (I). 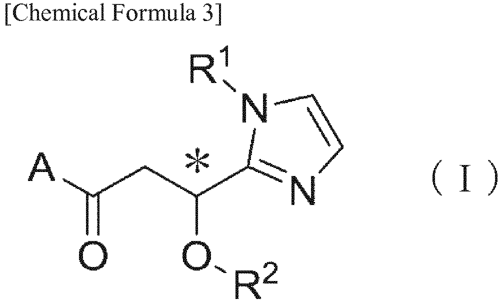 [wherein
carbon marked with * is asymmetric carbon, and A represents a group represented by general formulae (IIa), (IIb) or (IIc): 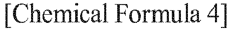 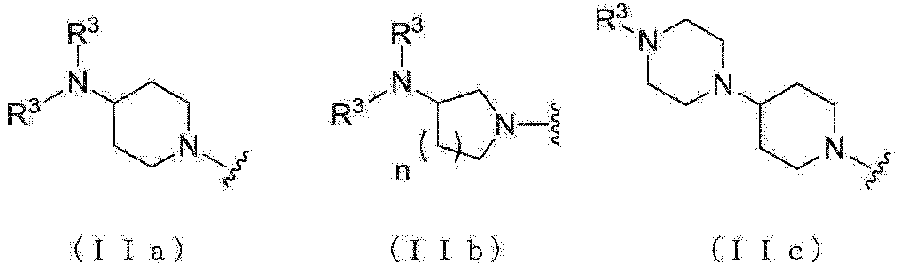 wherein R¹ represents a methyl group or an ethyl group optionally substituted with a halogen atom, R² represents a hydrogen atom or an alkylcarbonyl group having 2 to 5 carbon atoms, each R³ independently represents a methyl group or an ethyl group, and n represents 1 or 2.]

In the above cyclic amine derivative, it is preferable that A is the group represented by general formula (IIa), in which R¹ is preferably a methyl group or an ethyl group optionally substituted with a fluorine atom and more preferably a methyl group, an ethyl group, a difluoromethyl group or a 2,2,2-trifluoroethyl group.

In the above cyclic amine derivative, it is preferable that A is the group represented by general formulae (IIb) or (IIc), in which R¹ is preferably a methyl group or an ethyl group optionally substituted with a fluorine atom; and more preferably a methyl group, an ethyl group, a difluoromethyl group or a 2,2,2-trifluoroethyl group.

In the above cyclic amine derivative, it is preferable that the group represented by general formula (IIa) and that the stereochemical configuration of the asymmetric carbon marked with * is S, in which R¹ is preferably a methyl group or an ethyl group optinally substituted with a fluorine atom; and more preferably a methyl group, an ethyl group, a difluoromethyl group or a 2,2,2-trifluoroethyl group.

In an embodiment of the above cyclic amine derivative, A is the group represented by general formula (IIa), R¹ represents a methyl group, an ethyl group, a difluoromethyl group or a 2,2,2-trifluoroethyl group, R² represents a hydrogen atom or an alkylcarbonyl group having 2 to 5 carbon atoms and each R³ independently represents a methyl group or an ethyl group. In this embodiment, it is preferable that the stereochemical configuration of the asymmetric carbon marked with * is S.

In an embodiment of the above cyclic amine derivative, A is the group represented by general formula (IIa), R¹ represents a methyl group or a 2,2,2-trifluoroethyl group, R² represents a hydrogen atom or an alkylcarbonyl group having 2 carbon atoms and R³ represents a methyl group. In this embodiment, it is preferable that the stereochemical configuration of the asymmetric carbon marked with * is S.

In an embodiment of the above cyclic amine derivative, A is the group represented by general formula (IIb), R¹ represents a methyl group or an ethyl group which may be substituted with a fluorine atom, R² represents a hydrogen atom or an alkylcarbonyl group having 2 to 5 carbon atoms, each R³ represents independently a methyl group or an ethyl group, and n represents 1 or 2. In this embodiment, it is preferable that the stereochemical configuration of the asymmetric carbon marked with * is S.

In an embodiment of the above cyclic amine derivative, A is the group represented by general formula (IIb), R¹ represents a methyl group, an ethyl group, a difluoromethyl group or a 2,2,2-trifluoroethyl group, R² represents a hydrogen atom or an alkylcarbonyl group having 2 to 5 carbon atoms, each R³ represents independently a methyl group or an ethyl group, and n represents 1 or 2. In this embodiment, it is preferable that the stereochemical configuration of the asymmetric carbon marked with * is S.

In an embodiment of the above cyclic amine derivative, A is the group represented by general formula (IIb), R¹ represents a methyl group or a 2,2,2-trifluoroethyl group, R² represents a hydrogen atom or an alkylcarbonyl group having 2 carbon atoms, R³ represents a methyl group, and n represents 1 or 2. In this embodiment, it is preferable that the stereochemical configuration of the asymmetric carbon marked with * is S.

In an embodiment of the above cyclic amine derivative, A is the group represented by general formula (IIc), R¹ represents a methyl group or an ethyl group optionally substituted with a fluorine atom, R² represents a hydrogen atom or an alkylcarbonyl group having 2 to 5 carbon atoms, and R³ represents a methyl group or an ethyl group. In this embodiment, it is preferable that the stereochemical configuration of the asymmetric carbon marked with * is S.

In an embodiment of the above cyclic amine derivative, A is the group represented by general formula (IIc), R¹ represents a methyl group, an ethyl group, a difluoromethyl group or a 2,2,2-trifluoroethyl group, R² represents a hydrogen atom or an alkylcarbonyl group having 2 to 5 carbon atoms, and R³ represents a methyl group or an ethyl group. In this embodiment, it is preferable that the stereochemical configuration of the asymmetric carbon marked with * is S.

In an embodiment of the above cyclic amine derivative, A is the group represented by general formula (IIc), R¹ represents a methyl group or a 2,2,2-trifluoroethyl group, R² represents a hydrogen atom or an alkylcarbonyl group having 2 carbon atoms, and R³ represents a methyl group. In this embodiment, it is preferable that the stereochemical configuration of the asymmetric carbon marked with * is S.

The "halogen atom" refers to a fluorine atom, a chlorine atom, a bromine atom or an iodine atom.

The "methyl group or an ethyl group optionally substituted with a halogen atom" refers to a methyl group or an ethyl group in which hydrogen atoms are each independently and optionally substituted with a halogen atom as mentioned above. For example, a methyl group or an ethyl group, or a difluoromethyl group, a 2-fluoroethyl group, a 2-chloroethyl group, a 2,2-difluoroethyl group or a 2,2,2-trifluoroethyl group can be mentioned.

The "alkylcarbonyl group having 2 to 5 carbon atoms" refers to a group obtained by binding a linear, branched or cyclic saturated hydrocarbon group having 1 to 4 carbon atoms to a carbonyl group. For example, an acetyl group, a n-propionyl group, a n-butyryl group, an isobutyryl group or a valeryl group can be mentioned.

Specific examples of a preferable compound as a cyclic amine derivative represented by general formula (I) (hereinafter, cyclic amine derivative (I)) will be shown in Tables 1-1 and 1-2. However, the derivatives are not limited to these.

**[Table 1-1]**

| Structural formula | Structural formula | Structural formula |
|---|---|---|
| 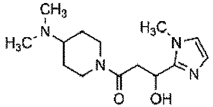 | 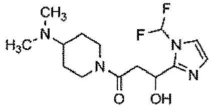 | 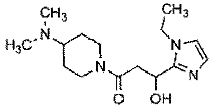 |
| 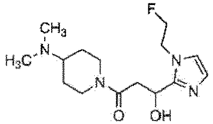 | 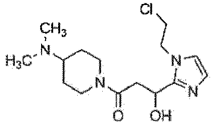 | 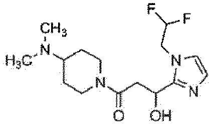 |
| 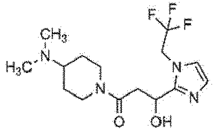 | 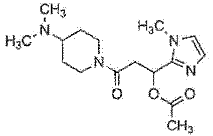 | 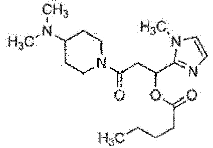 |
| 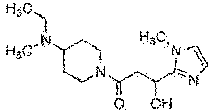 | 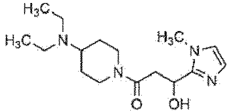 | 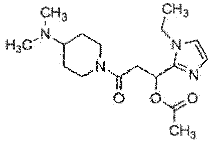 |
| 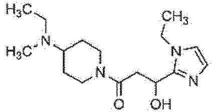 | 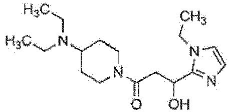 | 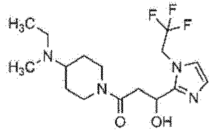 |
| 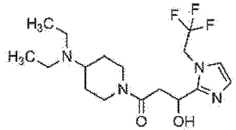 | 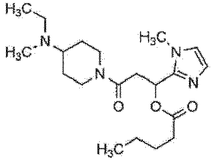 | 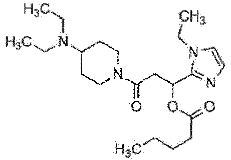 |
| 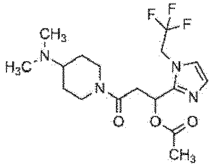 | 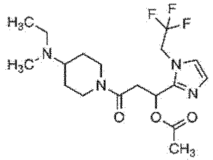 | 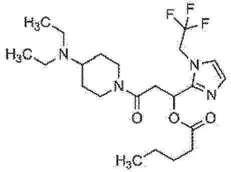 |

**[Table 1-2]**

| Structural formula | Structural formula | Structural formula |
|---|---|---|
| 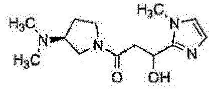 | 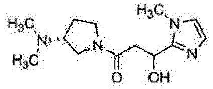 | 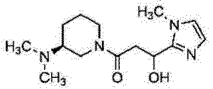 |
| 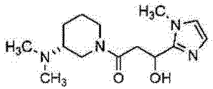 | 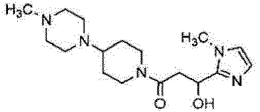 | 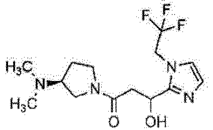 |
| 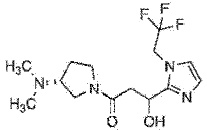 | 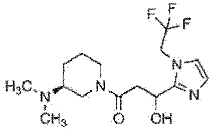 | 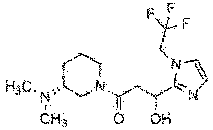 |
| 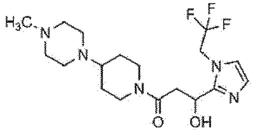 | 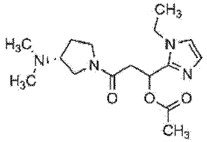 | 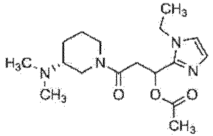 |
| 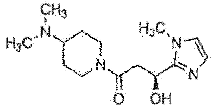 | 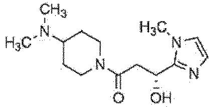 | 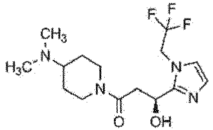 |
| 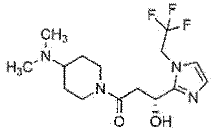 | 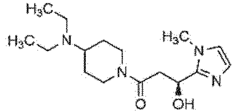 | 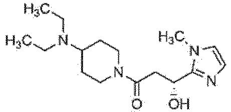 |
| 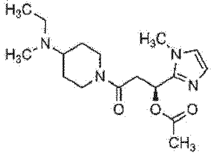 | 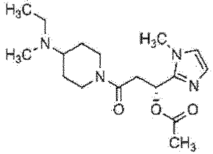 | 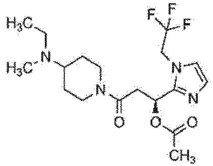 |
| 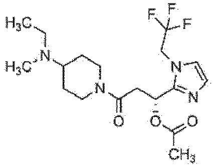 | 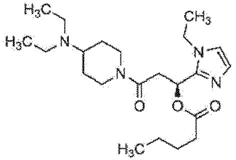 | 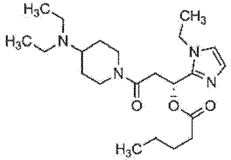 |

When the cyclic amine derivative (I) has isomers such as enantiomers and stereoisomers, any one of isomers and mixtures of these are included in the cyclic amine derivative (I). In addition, when the cyclic amine derivative (I) contains isomers such as enantiomers and stereoisomers, any one of isomers and mixtures of these are also included in the cyclic amine derivative (I). In addition, when conformational isomers are sometimes formed, such isomers and mixtures of these are also included in the cyclic amine derivative (I). A desired isomer can be obtained by a known method or a similar method thereto. For example, when an enantiomers of the cyclic amine derivative (I) is present, the enantiomer separated from the cyclic amine derivative (I) is also included in the cyclic amine derivative (I).

A desired enantiomer can be obtained by a known means (for example, an optically active synthetic intermediate is used or final-product racemic mixture is subjected to a known method or a similar method thereto (for example, optical resolution)).

A prodrug or a pharmacologically acceptable salt of the cyclic amine derivative (I) is also included. The prodrug of the cyclic amine derivative (I) refers to a compound, which is enzymatically or chemically converted to the cyclic amine derivative (I) *in vivo.* The active form of a prodrug of the cyclic amine derivative (I) is the cyclic amine derivative (I). However, a prodrug of the cyclic amine derivative (I) itself may have activity.

As the prodrug of the cyclic amine derivative (I), for example, a compound obtained by alkylation, phosphorylation or boration of a hydroxy group of the cyclic amine derivative (I) can be mentioned. These compounds can be each synthesized from the cyclic amine derivative (I) in accordance with a known method.

A prodrug of the cyclic amine derivative (I) may be converted into the cyclic amine derivative (I) in physiological conditions described in known literatures ("Development of pharmaceutical product", Hirokawa-Shoten Ltd., 1990, Vol. 7, p.163-198 and Progress in Medicine, Vol. 5, 1985, p.2157-2161).

The cyclic amine derivative (I) may be labeled with an isotope. Examples of isotopes for use in labeling include ²H, ³H, ¹³C, ¹⁴C, ¹⁵N, ¹⁵O, ¹⁸O and/or ¹²⁵I.

As the pharmacologically acceptable salt of the cyclic amine derivative (I), for example, an inorganic salt such as a hydrochloride, a sulfate, a phosphate or a hydrobromide; or organic salt such as an oxalate, a malonate, a citrate, a fumarate, a lactate, a malate, a succinate, a tartrate, an acetate, a trifluoroacetate, a maleate, a gluconate, a benzoate, a salicylate, a xinafoate, a pamoate, an ascorbate, an adipate, a methanesulfonate, a p-toluenesulfonate or a cinnamate. These salts may be present in the form of a hydrate, a solvate or a crystalline polymorph.

The cyclic amine derivative (I) or a pharmacologically acceptable salt can be synthesized in accordance with the method described in the known literature (International Publication WO2016/136944), for example.

Examples of peripheral nerves include sensory nerves, motor nerves and autonomic nerves.

Peripheral neuropathy is induced by damage to at least one of neurons and myelin sheaths (Schwann cells) constituting peripheral nerves.

Examples of peripheral neuropathies include, but are not limited to, drug-induced peripheral neuropathies, autoimmune peripheral neuropathies, metabolic peripheral neuropathies, hereditary peripheral neuropathies, vasculitic peripheral neuropathies, toxic peripheral neuropathies, infectious peripheral neuropathies, and peripheral neuropathies associated with malignant tumor.

Examples of the symptoms of peripheral neuropathies include, but are not limited to: when sensory nerves are damaged, numbness of limbs (dysesthesia), paresthesia, hypesthesia, pain, and hypacusia; when motor nerves are damaged, muscle weakness or atrophy, flaccid paralysis, and deep tendon reflex decrease or loss; and when autonomic nerves are damaged, constipation, abdominal pain, dyshidrosis, dysuria, and orthostatic hypotension.

Examples of drug-induced peripheral neuropathies include anticancer agent-induced peripheral neuropathy, antiviral agent-induced peripheral neuropathy, antimicrobial agent-induced peripheral neuropathy, antitubercular agent-induced peripheral neuropathy, antiarrhythmic agent-induced peripheral neuropathy, lipid-lowering drug-induced peripheral neuropathy, immunosuppressive drug-induced peripheral neuropathy, gout therapeutic agent-induced peripheral neuropathy, and peripheral neuropathies caused by other drugs.

Examples of anticancer agents include nucleic acid metabolism inhibitors, microtubule polymerization or depolymerization inhibitors, hormone antagonists, intracellular signaling inhibitors, malignant tumor specific molecular target drugs, and non-specific immunologic adjuvants.

Examples of nucleic acid metabolism inhibitors include alkylating agents, antineoplastic antibiotics, topoisomerase inhibitors, platinum drugs, pyrimidine metabolism inhibitors, purine metabolism inhibitors, and folic acid synthesis inhibitors.

Examples of microtubule polymerization or depolymerization inhibitors include vinca alkaloid anticancers drug and taxane anticancer drugs.

Examples of hormone antagonists include antiestrogens, and antiandrogens.

Examples of intracellular signaling inhibitors include proteosome inhibitors and cerebron inhibitors.

Examples of malignant tumor specific molecular target drugs include tyrosine kinase inhibitors, antibody formulations, and arsenic formulations.

Examples of non-specific immunologic adjuvants include hemolytic streptococcus formulations and Coriolus versicolor polysaccharide formulations.

Examples of nucleic acid metabolism inhibitors include, but are not limited to the following specific anticancer agents, oxaliplatin, cisplatin, carboplatin, nedaplatin, cytarabine, nelarabine, etoposide, and teniposide. Examples of microtubule polymerization or depolymerization inhibitors include, but are not limited to the following specific anticancer agents, paclitaxel, docetaxel, cabazitaxel, vincristine, vinblastine, vinorelbine, vindesine, eribulin, vinflunine, epothilone, and ixabepilone. Examples of intracellular signaling inhibitors include, but are not limited to the following specific anticancer agents, bortezomib and carfilzomib. Examples of malignant tumor specific molecular target drugs include, but are not limited to the following specific anticancer agents, brentuximab vedotin, trastuzumab emtansine, thalidomide, and pomalidomide or lenalidomide.

Examples of antiviral agents include, but are not limited to, efavirenz, emtricitabine, emtricitabine and tenofovir disoproxil fumarate, saquinavir, sanilvudine, zalcitabine, didanosine, stavudine, zidovudine, darunavir, delavirdine mesylate, nevirapine, tenofovir disoproxil fumarate, foscarnet sodium hydrate, lamivudine, lamivudine and abacavir sulfate, ritonavir, ribavirin, lopinavir and ritonavir, atazanavir, and indinavir.

Examples of antimicrobial agents include, but are not limited to, chloramphenicol, nitrofurantoin, metronidazole, diaphenylsulfone, ethambutol, and fluoroquinolone (levofloxacin, ciprofloxacin, moxifloxacin, norfloxacin, ofloxacin and the like).

Examples of antitubercular agents include, but are not limited to, isoniazid and ethambutol.

Examples of antiarrhythmic agents include, but are not limited to, amiodarone and procainamide.

Examples of lipid-lowering drugs include, but are not limited to, pravastatin, simvastatin, fluvastatin, atorvastatin, pitavastatin and rosuvastatin.

Examples of immunosuppressive drugs include, but are not limited to, tacrolimus, cyclosporin, mycophenolate mofetil, leflunomide, chloroquine, interferon α, and gold formulation.

Examples of other drugs include, but are not limited to, gout therapeutic agents such as colchicine or allopurinol, antiepileptic agents such as phenytoin, anesthetics such as nitrous oxide, vitamins such as pyridoxine, antialcoholic drugs such as disulfiram, and hypotensive drugs such as hydralazine.

Examples of drugs that induce drug-induced peripheral neuropathies include not only drugs that have been discovered to date, but also drugs that will be discovered in the future, on the basis of the above classification.

Examples of autoimmune peripheral neuropathies include, but are not limited to, Guillain-Barré syndrome, chronic inflammatory demyelinating polyradiculoneuropathy, multifocal motor neuropathy and paraproteinemic neuropathy. Examples of the subtypes of Guillain-Barré syndrome include acute inflammatory demyelinating polyneuropathy, acute motor axonal neurupathy, acute motor-sensory axonal neuropathy, and Fisher syndrome.

Examples of metabolic peripheral neuropathies include, but are not limited to, diabetic peripheral neuropathy, uremic peripheral neuropathy, collagen-peripheral neuropathy, vitamin deficiency peripheral neuropathy, and hypothyroidism peripheral neuropathy.

Examples of hereditary peripheral neuropathies include, but are not limited to, Charcot-Marie-Tooth disease, familial amyloid polyneuropathy, hereditary neuropathy to pressure palsies, and hereditary neuralgic amyotrophy.

Whether the cyclic amine derivative (I) or a pharmacologically acceptable salt thereof has an effect of suppressing damage to peripheral neurons can be evaluated using a rat dorsal root ganglion-derived established neuronal cell line. Specifically, the rat dorsal root ganglion-derived established neuronal cell line is treated with a cytotoxic substance to induce a decrease in cell activity, and then the effect of suppressing such a decrease in cell viability is evaluated.

Whether the cyclic amine derivative (I) or a pharmacologically acceptable salt thereof has an effect of accelerating myelination can be evaluated using co-culture of rat dorsal root ganglion neurons and Schwann cells. Specifically, rat dorsal root ganglion neurons and Schwann cells are cultured together, myelination is induced by ascorbic acid treatment and then if myelination is accelerated is evaluated.

The above derivative or the pharmacologically acceptable salt thereof having the above effects of suppressing decreases in cell viability and accelerating myelination is considered to be effective for prevention and treatment of peripheral neuropathies. However, the embodiment is not limited by the assumption.

Whether the cyclic amine derivative (I) or a pharmacologically acceptable salt thereof is effective for treatment or prevention of drug-induced peripheral neuropathies, and particularly anticancer agent-induced peripheral neuropathy, can be evaluated using models of peripheral neuropathies induced by various drugs, and particularly various anticancer agents (Hoeke et al. ILAR Journal, 2014, Vol. 54, p. 273-281).

Whether the cyclic amine derivative (I) or a pharmacologically acceptable salt thereof is effective for treatment or prevention of autoimmune peripheral neuropathies can be evaluated using an experimental autoimmune neuritis (EAN) model (Soliven, ILAR Journal, 1994, Vol. 54, p. 282-290).

Whether the cyclic amine derivative (I) or a pharmacologically acceptable salt thereof is effective for treatment or prevention of metabolic peripheral neuropathies, and particularly diabetic peripheral neuropathy, can be evaluated using a streptozotocin-induced diabetes model (O'Brien et al. ILAR Journal, 2014, Vol. 54, p. 259-272).

Whether the cyclic amine derivative (I) or a pharmacologically acceptable salt thereof is effective for treatment or prevention of hereditary peripheral neuropathies, and particularly Charcot-Marie-Tooth disease, can be evaluated using PMP22 Trembler-J mice (Nicks et al. Neurobiology of Disease, 2014, Vol. 70, p. 224-236).

The cyclic amine derivative (I) or a pharmacologically acceptable salt thereof can be used as an excellent pharmaceutical product useful for treatment or prevention of peripheral neuropathies in mammals (for example, mouse, rat, hamster, rabbit, cat, dog, cow, sheep, monkey or human), and especially to a human.

When the cyclic amine derivative (I) or a pharmacologically acceptable salt thereof is used as a medicine, the cyclic amine derivative (I) or a pharmacologically acceptable salt thereof directly or in combination with a pharmaceutically acceptable carrier can be orally or parenterally administered.

As the dosage form when a medicine containing the cyclic amine derivative (I) or a pharmacologically acceptable salt thereof as an active ingredient is orally administered, for example, tablets (including sugar-coated and film-coated tablets), pills, granules, powders, capsules (including soft capsules and micro capsules), syrups, emulsions or suspensions can be mentioned. As the dosage form when a medicine containing the cyclic amine derivative (I) or a pharmacologically acceptable salt thereof as an active ingredient is parenterally administered, for example, injections, infusions, drops, suppositories, endermic liniments or adhesive patches can be mentioned. It is further effective to prepare a sustained-relase formulation by using an appropriate base (for example, a butyric acid polymer, a glycolic acid polymer, a butyric acid-glycolic acid copolymer, mixtures of a butyric acid polymer and a glycolic acid polymer, or a polyglycerol fatty acid ester) in combination.

Formulations having the aforementioned dosage forms can be prepared in accordance with production methods known in the field of drug formulation. In this case, if necessary, production can be made by adding an excipient, a binder, a lubricant, a disintegrating agent, a sweetening agent, a surfactant, a suspending agent or an emulsifying agent, which is generally used in the field of drug formulation.

Tablets can be prepared, for example, by adding an excipient, a binder, a disintegrating agent or a lubricant. Pills and granules can be prepared by adding, for example, an excipient, a binder or a disintegrating agent. Powders and capsules can be prepared by adding, for example, an excipient. Syrups can be prepared by adding, for example, a sweetening agent. Emulsions or suspensions can be prepared by adding, for example, a surfactant, a suspending agent or an emulsifier.

As the excipient, for example, lactose, glucose, starch, sucrose, microcrystalline cellulose, powdered *glycyrrhiza,* mannitol, sodium hydrogen carbonate, calcium phosphate or calcium sulfate can be mentioned.

As the binder, for example, a starch paste solution, a gum arabic solution, a gelatin solution, a tragacanth solution, a carboxymethylcellulose solution, a sodium alginate solution or glycerin can be mentioned.

As the disintegrating agent, for example, starch or calcium carbonate can be mentioned.

As the lubricant, for example, magnesium stearate, stearic acid, calcium stearate or purified talc can be mentioned.

As the sweetening agent, for example, glucose, fructose, inverted sugar, sorbitol, xylitol, glycerin or simple syrup can be mentioned.

As the surfactant, for example, sodium lauryl sulfate, polysorbate 80, sorbitan monofatty acid ester or stearic acid polyoxyl 40 can be mentioned.

As the suspending agent, for example, Gum arabic, sodium alginate, sodium carboxymethylcellulose, methylcellulose or bentonite can be mentioned.

As the emulsifier, for example, Gum arabic, tragacanth, gelatin or polysorbate 80 can be mentioned.

When a medicine containing the cyclic amine derivative (I) or a pharmacologically acceptable salt thereof as an active ingredient is prepared in the aforementioned dosage forms, a coloring agent, a preserving agent, a fragrance, a flavoring agent, a stabilizer or thickener generally used in the field of drug formulation can be added.

The dose per day of a medicine containing the cyclic amine derivative (I) or a pharmacologically acceptable salt thereof as an active ingredient varies depending upon e.g., the state or body weight of the patient or the type or administration route of a compound. For example, in oral adminstration to an adult (weight: about 60 kg), the amount of the cyclic amine derivative (I) or a pharmacologically acceptable salt thereof serving as an active ingredient falls within the range of 1 to 1000 mg and administration is preferably made in 1 to 3 divided doses. For example in parental administration to an adult (weight: about 60 kg) by an injectable solution, the amount of the cyclic amine derivative (I) or a pharmacologically acceptable salt thereof serving as an active ingredient in e.g., an injection, falls within the range of 0.01 to 100 mg per body weight (1 kg). The injectable solution is preferably intravenous administered.

The cyclic amine derivative (I) or a pharmacologically acceptable salt thereof may be used in combination with other medical agents in an appropriate blending ratio to supplement or enhance a therapeutic or prophylactic effect or reduce the dose. For example, the cyclic amine derivative (I) or a pharmacologically acceptable salt thereof can also be used in combination with a drug for relieving the symptoms of peripheral neuropathies.

### EXAMPLES

Hereinafter, the present invention will be described more specifically with reference to Examples. However, the present invention is not limited by these Examples.

As a test compound, (S)-1-(4-(dimethylamino)piperidin-1-yl)-3-hydroxy-3-(1-methyl-1H-imidazol-2-yl)propan-1-one (hereinafter, compound 1), which is represented by the following formula, is used and synthesized according to the method described in the known literature (International Publication WO2016/136944).

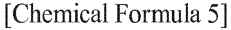

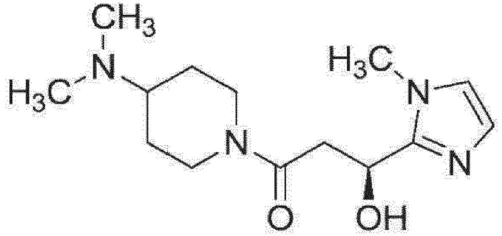

(Example 1) Protective effect of the cyclic amine derivative (I) or a pharmacologically acceptable salt thereof on cell damage in rat dorsal root ganglion-derived established neuronal cell line:
The protective effect of the cyclic amine derivative (I) or a pharmacologically acceptable salt thereof on cell damage in a rat dorsal root ganglion-derived established neuronal cell line was investigated.

A fetal rat dorsal root ganglion-derived established neuronal cell line ND15 was cultured in 10% FBS-containing DMEM. On the next day, the cells were cultured in 10% FBS-containing DMEM containing EC23 (10 µM, Reinner) for 8 days, for differentiation into neurons.

The medium was exchanged with DMEM/F12 medium containing cisplatin (final concentration of 50 µM) followed by 4 hours of culture, thereby inducing cell injury. Compound 1 was contained in the medium (final concentration of 0.5, 5 or 50 µM) for treatment, in a manner similar to that for cisplatin. Groups were composed of 6 groups: an untreated group, a 50 µM compound 1 treatment group (the group treated with 50 µM compound 1), a cisplatin treatment group, a cisplatin and 0.5 µM compound 1 treatment group (the group treated with cisplatin and 0.5 µM compound 1), a cisplatin and 5 µM compound 1 treatment group, and a cisplatin and 50 µM compound 1 treatment group.

For measurement of cell viability, the medium was exchanged with DMEM/F12 medium containing alamarBlue (Invitrogen), followed by 2 hours of culture, and then the absorbance at wavelength of 570 nm and the absorbance at wavelength of 595 nm were measured. Cell viability was calculated using the ratio of the absorbance at wavelength of 570 nm to the absorbance at wavelength of 595 nm and the untreated group designated as 100%.

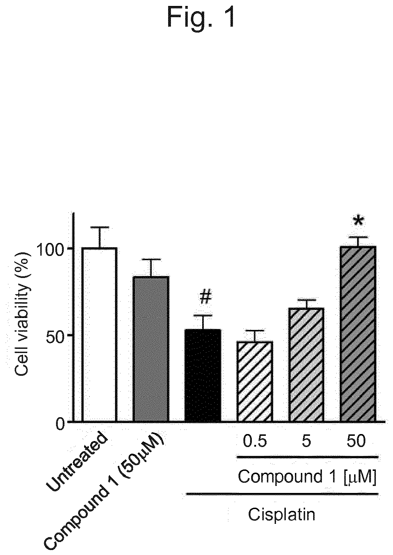

The results of evaluating the effects of compound 1 on cell viability are shown in Figure 1. In Figure 1, the vertical axis indicates cell viability (%) (mean value ± standard error; n = 6 per group), and the horizontal axis indicates, from the left, the untreated group, the 50 µM compound 1 treatment group, the cisplatin treatment group, the cisplatin and 0.5 µM compound 1 treatment group, the cisplatin and 5 µM compound 1 treatment group, and the cisplatin and 50 µM compound 1 treatment group. The symbol "#" in Figure 1 indicates a statistically significant (#: p<0.05, Student's t-test) difference compared with the untreated group, and the symbol "*" in Figure 1 indicates a statistically significant (*: p<0.025, Williams' multiple comparison, one sided) difference compared with the cisplatin treatment group.

While a decrease in cell viability was observed in the rat dorsal root ganglion-derived established neuronal cell line as a result of treatment with cisplatin, the suppressed decrease in the same was observed as a result of simultaneous treatment with compound 1. Specifically, it was revealed that compound 1 protects the rat dorsal root ganglion-derived established neuronal cell line from being damaged.

(Example 2) Repair effect of the cyclic amine derivative (I) or a pharmacologically acceptable salt thereof on cell damage in rat dorsal root ganglion-derived established neuronal cell line:
The repair effect of the cyclic amine derivative (I) or a pharmacologically acceptable salt thereof on cell injury in a rat dorsal root ganglion-derived established neuronal cell line was investigated.

In a manner similar to Example 1, cell damage was induced to a fetal rat dorsal root ganglion-derived established neuronal cell line ND15 by the use of cisplatin, and then the cell viability was measured. After 24 hours of treatment with cisplatin, compound 1 was contained in a medium for 2 hours of treatment (final concentration of 0.5, 5 or 50 µM). Groups were composed of 6 groups: an untreated group; a 50 µM compound 1 treatment group; a cisplatin treatment group; a cisplatin and 0.5 µM compound 1 treatment group; a cisplatin and 5 µM compound 1 treatment group; and a cisplatin and 50 µM compound 1 treatment group.

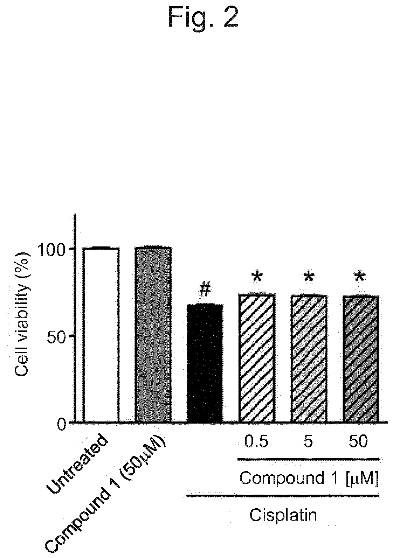

The results of evaluating the effects of compound 1 on cell viability are shown in Figure 2. In Figure 2, the vertical axis indicates cell viability (%) (mean value ± standard error; n = 6 per group). The horizontal axis indicates, from the left, the untreated group, the 50 µM compound 1 treatment group, the cisplatin treatment group, the cisplatin and 0.5 µM compound 1 treatment group, the cisplatin and 5 µM compound 1 treatment group, and the cisplatin and 50 µM compound 1 treatment group. The symbol "#" in Figure 2 indicates a statistically significant (#: p<0.05, Student's t-test) difference compared with the untreated group, and the symbols "*" in Figure 2 indicate statistically significant (*: p<0.025, Williams' multiple comparison, one sided) differences compared with the cisplatin treatment group.

While decreases in cell viability were observed in the rat dorsal root ganglion-derived established neuronal cell line as a result of treatment with cisplatin, the suppressed decrease in the same was observed as a result of post-treatment with compound 1. Specifically, it was revealed that compound 1 repairs damage to the rat dorsal root ganglion-derived established neuronal cell line.

(Example 3) Effects of the cyclic amine derivative (I) or a pharmacologically acceptable salt thereof on myelination in co-culture of rat dorsal root ganglion neurons and Schwann cells:
The accelerating effect of the cyclic amine derivative (I) or a pharmacologically acceptable salt thereof on myelination in the co-culture of rat dorsal root ganglion neurons and Schwann cells was studied.

The dorsal root ganglion was excised from a fetus of a female SD rat at 15 days of pregnancy, and then neurons and Schwann precursor cells thereof were separately cultured (the initial day of cell culture was designated as Day 1). On day 19 from the initial day of cell culture (Day 19), Schwann precursor cells were added to the culture of neurons to initiate co-culture. Myelination was induced by treatment with ascorbic acid (a total of 4 to 5 times in accordance with 2 to 3 times of medium exchange) on the 26^{th} to 40^{th} days (Days 26 to 40) from the initial day of cell culture.

In combination with ascorbic acid treatment, treatment with compound 1 dissolved in sterile distilled water (final concentration of 30 µM) was performed 4 to 5 times in total. As a control, treatment with sterile distilled water instead of the compound 1 solution was performed. Groups were composed of 2 groups: a sterile distilled water treatment group (Vehicle treatment group); and a 30 µM compound 1 treatment group (compound 1 treatment group).

For immunostaining, cells were washed with phosphate-buffered saline on the 40^{th} to 43^{rd} days (Days 40 to 43) from the initial day of cell culture, and then fixed with 4% paraformaldehyde in phosphate buffer. After methanol treatment and blocking, a myelin sheath marker protein, myelin basic protein (MBP), was subjected to immunofluorescence staining.

Fluorescent images of MBP were taken with a fluorescence microscope (DMI4000B, Leica), and then the number of myelin sheath segments (≥25 µm) was analyzed based on the images. Note that the analysis region of interest (ROI) was a portion where the highest number of fibrous stained images of MBP was observed among 4 portions prepared by dividing the area of co-culture by four.

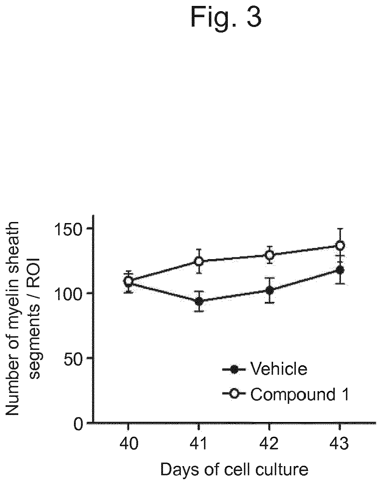

The results of evaluating the effects of compound 1 on myelination are shown in Figure 3. In Figure 3, the vertical axis indicates the number of myelin sheath segments per ROI (mean value ± standard error; n = 8 to 12 per group), and horizontal axis indicates the number of days of cell culture.

(Example 4) Effects of the cyclic amine derivative (I) or a pharmacologically acceptable salt thereof on expression level of MBP in co-culture of rat dorsal root ganglion neurons and Schwann cells:
The effects of the cyclic amine derivative (I) or a pharmacologically acceptable salt thereof on expression level of MBP in the co-culture of rat dorsal root ganglion neurons and Schwann cells were investigated.

In a manner similar to Example 3, co-culture of rat dorsal root ganglion neurons and Schwann cells was prepared, myelination was induced, and then treatment with compound 1 was performed. Groups were composed of 2 groups: a sterile distilled water treatment group (Vehicle treatment group); and a 30 µM compound 1 treatment group (compound 1 treatment group).

For Western blotting, on day 43 from the initial day of cell culture, the co-culture was lysed in a RIPA cell lysis solution, and then the cell lysate was subjected to sodium dodecyl sulfate-polyacrylamide gel electrophoresis. Subsequently, the protein was transferred to a PVDF membrane, and then an MBP band was detected by antibody reaction. Image Lab software (BIO-RAD) was used for quantifying the thus detected band.

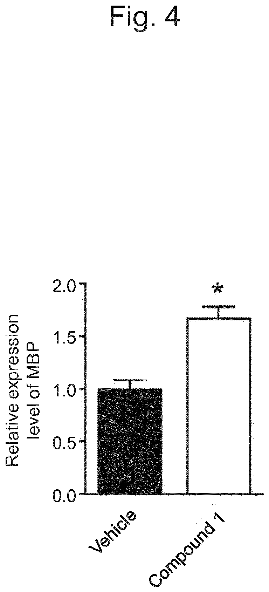

The results of evaluating the effects of compound 1 on expression level of MBP are shown in Figure 4. In Figure 4, the vertical axis indicates relative expression levels of MBP to the mean value of the vehicle group designated as 1 (mean value ± standard error; n = 4 per group), and the horizontal axis indicates, from the left, the vehicle treatment group and the compound 1 treatment group. The symbol "*" in Figure 4 indicates a statistically significant (*: p<0.05, Student's t-test) difference compared with the vehicle treatment group.

Increased number of myelin sheath segments and increases in the myelin sheath marker protein, MBP, were observed as a result of treatment with compound 1. Specifically, it was revealed that compound 1 accelerates myelination in the co-culture of rat dorsal root ganglion neurons and Schwann cells.

As revealed in Examples 1, 2, 3 and 4, the cyclic amine derivative (I) or a pharmacologically acceptable salt thereof is effective against damages to neurons and myelin sheaths of the peripheral nerves.

(Example 5) Effects of repeated dosing of the cyclic amine derivative (I) or a pharmacologically acceptable salt thereof on allodynia to cold stimuli (cold allodynia) and allodynia to tactile stimuli (mechanical allodynia) in rat oxaliplatin-induced peripheral neuropathy model:
The effects of the cyclic amine derivative (I) or a pharmacologically acceptable salt thereof on cold allodynia and mechanical allodynia which are developed by the administration of oxaliplatin were invetigated.

Oxaliplatin (4 mg/kg, Elplat I.V. infusion solution 200 g; Yakult) was administered to SD rats (7 weeks old, male; CHARLES RIVER LABORATORIES JAPAN, INC.) in a cycle of 1 week (intraperitoneally administered twice, two consecutive days per week) for 2 or 3 weeks, thereby preparing an oxaliplatin-induced peripheral neuropathy model. As a control (pseudo-induction), a 5% glucose solution (Otsuka Pharmaceutical Factory, Inc.) was administered. The initial day of administration was designated as day 0 after induction of pathological conditions.

A solution containing compound 1 (3 or 10 mg/kg) or a solvent thereof (water for injection; Otsuka Pharmaceutical Factory, Inc.) was orally administered everyday (twice a day) (2^{nd} administration was performed 8 hours after the 1^{st} administration) to rats from day 0 after induction of pathological conditions for 18 days. On day 0 after induction of pathological conditions, a 1^{st} administration was performed before administration of oxaliplatin, and on the day of evaluation of allodynia, a 1^{st} administration was performed after evaluation. Groups were composed of 4 groups: a pseudo-induction and solvent administration group (sham group); a pathological condition-induced and solvent administration group (vehicle group); a pathological condition-induced and compound 1 (3 mg/kg) administration group (compound 1 (3 mg/kg) group); and a pathological condition-induced and compound 1 (10 mg/kg) administration group (compound 1 (10 mg/kg) group).

The beneficial effects on cold allodynia were evaluated before induction of pathological conditions and on day 12 after induction of pathological conditions (before the 1^{st} administration of compound 1). Beneficial effects on cool allodynia were evaluated by cold plate test. A cold plate apparatus (Ugo Basile) was used for the test. An animal was placed on a plate kept at a predetermined temperature (8°C), and then the withdrawal latency required until pain-related behaviors (hind paw liftng, hind paw shaking, hind paw licking, standing, or jumping) were confirmed was measured. Note that the cut off time was designated as 180 seconds.

Beneficial effects on mechanical allodynia were evaluated before induction of pathological conditions and on day 18 after induction of pathological conditions (before the 1^{st} administration of compound 1). Beneficial effects on mechanical allodynia were evaluated by von Frey test. Note that the test method was performed according to the method described in the known literature (Chaplan et al. Journal of Neuroscience Methods, 1994, Vol. 53, p. 55-63) using von Frey filaments (North Coast Medical), so that 50% response threshold was calculated.

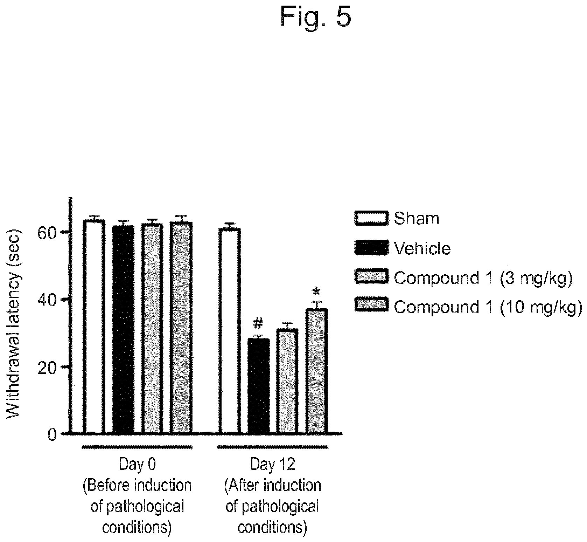

The results of evaluating the effects of compound 1 on cold allodynia are shown in Figure 5. In Figure 5, the vertical axis indicates withdrawal latencies in the cold plate test, and the higher numerical value indicates that cold allodynia is improved (mean value ± standard error; n = 10 per group). The horizontal axis indicates, from the left, each group before induction of pathological conditions (in Figure 5, "Day 0 (before induction of pathological conditions)"), and each group on day 12 after induction of pathological conditions (in Figure 5, "Day 12 (after induction of pathological conditions)"). The symbol "#" in Figure 5 indicates a statistically significant (#: p<0.05, Student's t-test) difference compared with the sham group, and the symbol "*" in Figure 5 indicates a statistically significant (*: p<0.025, Williams' multiple comparison, one sided) difference compared with the vehicle group.

On day 12 after induction of pathological conditions, a significant reduction in withdrawal latency was observed in the vehicle group, compared with the sham group. Specifically, the development of cold allodynia which is a peripheral neuropathy induced by oxaliplatin was confirmed.

Through daily (twice a day) oral administration of compound 1, a significantly prolonged withdrawal latency was observed in the compound 1 (10 mg/kg) group on day 12 after induction of pathological conditions, compared with the vehicle group. Specifically, it was revealed that compound 1 suppresses cold allodynia in the oxaliplatin-induced peripheral neuropathy model.

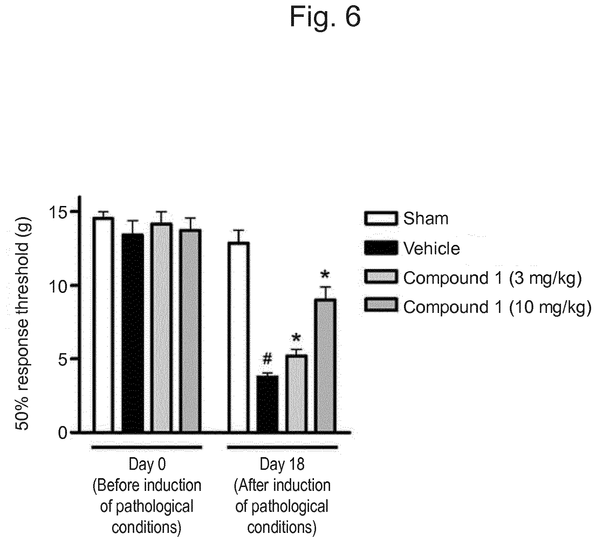

The results of evaluating the effects of compound 1 on mechanical allodynia are shown in Figure 6. In Figure 6, the vertical axis indicates 50% response thresholds in von Frey test, and the higher numerical value indicates that mechanical allodynia is improved (mean value ± standard error; n = 10 per group). The horizontal axis indicates, from the left, each group before induction of pathological conditions (in Figure 6, "Day 0 (before induction of pathological conditions)"), and each group on day 18 after induction of pathological conditions (in Figure 6, "Day 18 (after induction of pathological conditions)"). The symbol "#" in Figure 6 indicates a statistically significant (#: p<0.05, Welch's t-test) difference compared with the sham group, and the symbols "*" in Figure 6 indicate statistically significant (*: p<0.025, Shirley-Williams' multiple comparison, one sided) differences compared with the vehicle group.

On day 18 after induction of pathological conditions, a significant decrease in 50% response threshold was observed in the vehicle group, compared with the sham group. Specifically, the development of mechanical allodynia which is a peripheral neuropathy induced by oxaliplatin was confirmed.

Through daily (twice a day) oral administration of compound 1, a significant increase in 50% rensponse threshold was observed in the compound 1 (3 mg/kg) group and the compound 1 (10 mg/kg) group on day 18 after induction of pathological conditions, compared with the vehicle group. Specifically, it was revealed that compound 1 suppresses mechanical allodynia in the oxaliplatin-induced peripheral neuropathy model.

As described above, it was revealed that the cyclic amine derivative (I) or a pharmacologically acceptable salt thereof exhibits a significant suppressive effect on the peripheral neuropathy induced by oxaliplatin.

(Example 6) Therapeutic effect of single dosing of the cyclic amine derivative (I) or a pharmacologically acceptable salt thereof on mechanical allodynia in rat cisplatin-induced peripheral neuropathy model:
The effects of the cyclic amine derivative (I) or a pharmacologically acceptable salt thereof on mechanical allodynia developed by administration of cisplatin were investigated.

Cisplatin (Wako Pure Chemical Industries, Ltd.) was intraperitoneally administered to SD rats (6 weeks old, male; CHARLES RIVER LABORATORIES JAPAN, INC.) intermittently twice a week (1 or 2 mg/kg) for 5 weeks, thereby preparing a cisplatin-induced peripheral neuropathy model. Cisplatin was dissolved in physiological saline to a concentration of 10 mg/mL and then administered. As a control (pseudo-induction), physiological saline was administered. The initial day of administration was designated as day 1 after induction of pathological conditions.

On day 34 after induction of pathological conditions, a solution containing compound 1 (10 mg/kg) or a solvent thereof (water for injection) was orally administered to rats. Groups were composed of 3 groups: a pseudo-induction and solvent administration group (sham group); a pathological condition-induced and solvent administration group (vehicle group); and a pathological condition-induced and compound 1 (10 mg/kg) administration group (compound 1 (10 mg/kg) group).

Beneficial effects on mechanical allodynia were evaluated in a manner similar to Example 5 and this evaluation was performed before and 2 hours after administration of compound 1 on day 34 after induction of pathological conditions.

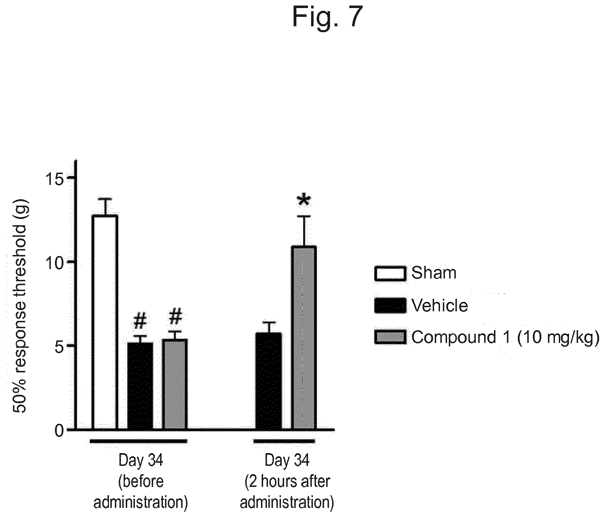

The results of evaluating the effects of compound 1 on mechanical allodynia are shown in Figure 7. In Figure 7, the vertical axis indicates 50% response thresholds in von Frey test and the higher numerical value indicates that mechanical allodynia is improved (mean value ± standard error; n = 4 to 6 per group), and the horizontal axis indicates, from the left, each group before administration of compound 1 (in Figure 7, "Day 34 (before administration)"), and each group at 2 hours after administration of the compound (in Figure 7, "Day 34 (2 hours after administration)"). The symbols "#" in Figure 7 indicate statistically significant (#: p<0.05, Student's t-test) differences compared with the sham group, and the symbol "*" in Figure 7 indicates a statistically significant (*: p<0.05, Student's t-test) difference compared with the vehicle group.

Two hours after administration of compound 1, a significant increase in 50% response threshold was observed in the compound 1 (10 mg/kg) group, compared with the vehicle group. Specifically, it was revealed that compound 1 suppresses mechanical allodynia in the cisplatin-induced peripheral neuropathy model.

As described above, it was revealed that the cyclic amine derivative (I) or a pharmacologically acceptable salt thereof exhibits a significant suppressive effect on the peripheral neuropathy induced by cisplatin.

(Example 7) Effects of single dose of the cyclic amine derivative (I) or a pharmacologically acceptable salt thereof on mechanical allodynia in rat paclitaxel-induced peripheral neuropathy model:
The effects of the cyclic amine derivative (I) or a pharmacologically acceptable salt thereof on mechanical allodynia developed by administration of paclitaxel were investigated.

Paclitaxel (4 mg/kg, ChromaDex) was intraperitoneally administered to SD rats (6 weeks old, male; CHARLES RIVER LABORATORIES JAPAN, INC.) every other day for 4 times, thereby preparing a paclitaxel-induced peripheral neuropathy model. Paclitaxel was dissolved in Cremophor EL (NACALAI TESQUE, INC.) and ethanol (Wako Pure Chemical Industries, Ltd.) mixed at 1:1 to a concentration of 6 mg/mL. The thus prepared solution was diluted with physiological saline to a concentration of 4 mg/mL and then administered. The initial day of administration was designated as day 0 after induction of pathological conditions.

On day 14 after induction of pathological conditions, a solution containing compound 1 (10 mg/kg) or a solvent thereof (water for injection) was orally administered to rats. Groups were composed of 2 groups: a solvent administration group (vehicle group) and a compound 1 (10 mg/kg) administration group.

Beneficial effects on mechanical allodynia were evaluated in a manner similar to Example 5, and the evaluation was performed before and on day 14 after induction of pathological conditions (3 hours after administration of compound 1).

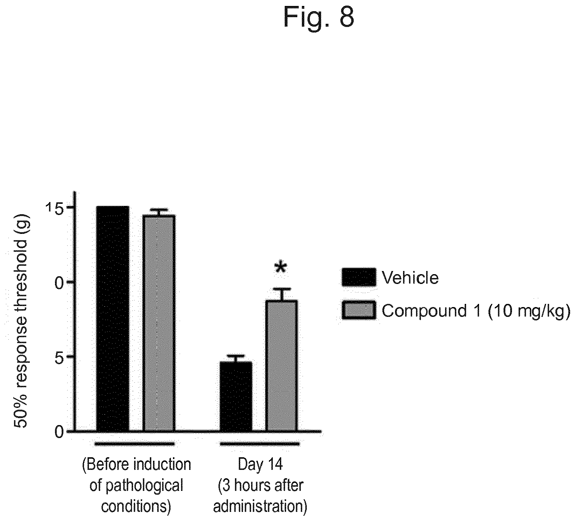

The results of evaluating the effects of compound 1 on mechanical allodynia are shown in Figure 8. In Figure 8, the vertical axis indicates 50% response thresholds in von Frey test and the higher numerical value indicates that mechanical allodynia is improved (mean value ± standard error; n = 8 per group). The horizontal axis indicates, from the left, each group before induction of pathological conditions, and each group at 3 hours after administration of compound 1 on day 14 after induction of pathological conditions (In Figure 8, "Day 14 (3 hours after administration)"). The symbol "*" in Figure 8 indicates a statistically significant (*: p<0.05, Student's t-test, two sided) difference compared with the vehicle group.

On day 14 after induction of pathological conditions (3 hours after administration of compound 1), a significant decrease in 50% renponse threshold was observed in the compound 1 (10 mg/kg) group, compared with the vehicle group. Specifically, it was revealed that compound 1 suppresses mechanical allodynia in the paclitaxel-induced peripheral neuropathy model.

As described above, it was revealed that the cyclic amine derivative (I) or a pharmacologically acceptable salt thereof exhibits a significant suppressive effect on the peripheral neuropathy induced by paclitaxel.

(Example 8) Effects of single dose of the cyclic amine derivative (I) or a pharmacologically acceptable salt thereof on mechanical allodynia in rat bortezomib-induced peripheral neuropathy model:
The therapeutic effect of the cyclic amine derivative (I) or a pharmacologically acceptable salt thereof on mechanical allodynia developed by administration of bortezomib was studied.

Bortezomib (0.2 mg/kg, AdooQ BioScience) was intraperitoneally administered 4 times in total to SD rats (6 weeks old, male; CHARLES RIVER LABORATORIES JAPAN, INC.) on days 1, 4, 8 and 11 after induction of pathological conditions, thereby preparing a bortezomib-induced peripheral neuropathy model. Here, the initial day of administration was designated as the 1^{st} day (Day1) after induction of pathological conditions. Bortezomib was dissolved in dimethyl sulfoxide, and then Tween80 was added. Subsequently, water for injection was added, so as to prepare a 0.2 mg/mL solution. The final concentration of dimethyl sulfoxide and that of Tween80 were each 5%.

On day 15 after induction of pathological conditions, a solution containing compound 1 (20 mg/kg) or a solvent thereof (water for injection) was orally administered to rats. Groups were composed of 2 groups: a solvent administration group (vehicle group) and a compound 1 (20 mg/kg) administration group.

Beneficial effects on mechanical allodynia were evaluated in a manner similar to Example 5, and this evaluation was performed before and on day 15 after induction of pathological conditions (3 hours after administration of compound 1).

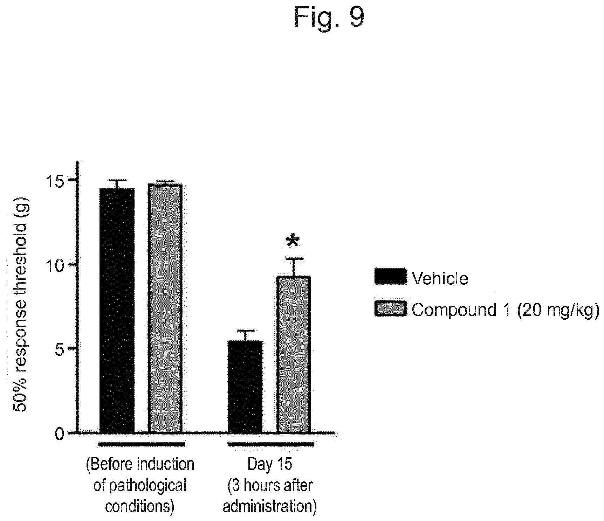

The results of evaluating the effects of compound 1 on mechanical allodynia are shown in Figure 9. In Figure 9, the vertical axis indicates 50% response thresholds in von Frey test, and the higher numerical value indicates that mechanical allodynia is improved (mean value ± standard error; n = 8 per group). The horizontal axis indicates, from the left, each group before induction of pathological conditions, and each group at 3 hours after administration of compound 1 on day 15 after induction of pathological conditions (in Figure 9, "Day 15 (3 hours after administration)"). The symbol "*" in Figure 9 indicates a statistically significant (*: p<0.05, Student's t-test, two sided) difference compared with the vehicle group.

On day 15 after induction of pathological conditions (3 hours after administration of compound 1), a significant increase in 50% response threshold was observed in the compound 1 (20 mg/kg) group, compared with the vehicle group. Specifically, it was revealed that compound 1 suppresses mechanical allodynia in the Bortezomib-induced peripheral neuropathy model.

As described above, it was revealed that the cyclic amine derivative (I) or a pharmacologically acceptable salt thereof exhibits a significant suppressive effect on the peripheral neuropathy induced by bortezomib.

Therefore, it was revealed that the cyclic amine derivative (I) or a pharmacologically acceptable salt thereof exhibits a significant suppressive effect on peripheral neuropathies induced by drugs, particularly anticancer agents.

(Example 9) Effects of the cyclic amine derivative (I) or a pharmacologically acceptable salt thereof on rat experimental autoimmune neuritis (EAN) model:
The suppressive effect of the cyclic amine derivative (I) or a pharmacologically acceptable salt thereof on a rat EAN model was investigated.

A method for preparing EAN model rats is as described below. Peripheral myelin protein P2 peptide 57-81 (synthesized at Toray Research Center, Inc.) was dissolved in physiological saline (Otsuka Pharmaceutical Factory, Inc.) to prepare a 2 mg/mL solution. The solution and 2 mg/mL complete Freund's adjuvant (Difco Laboratories) containing killed *Mycobacterium tuberculosis* H37Ra were mixed in equal amounts, thereby preparing an emulsion which was a peptide solution for administration. The peptide solution for administration (200 µL) was administered subcutaneously to the base of the tail of each Lewis rat (6 to 7 weeks old, male; CHARLES RIVER LABORATORIES JAPAN, INC.) under anesthesia, thereby preparing the EAN model. The day of administration of the peptide was designated as day 0 after induction of pathological conditions.

Clinical scores were evaluated as follows: 0 = no symptom, 1 = limp tail or hind limb weakness, 2 = limp tail and hind limb weakness, 3 = partial hind limb paralysis, 4 = complete hind limb paralysis, 5 = moribund state or death. Weighing was also performed.

Compound 1 (20 mg/kg) was dissolved in distilled water (Otsuka Pharmaceutical Factory, Inc.) and then the solution was orally administered to the EAN model twice a day (administration was started on day 10 after induction of pathological conditions). As a control, distilled water was orally administered to the EAN model. Groups were composed of 2 groups: a solvent administration group (vehicle group); and a compound 1 administration group.

For histopathological evaluation, on day 17 after induction of pathological conditions, the sciatic nerve and the tibial nerve were isolated, and then immersed in a 10% formalin neutral buffer solution. Specimens were sliced and then subjected to hematoxylin-eogin staining, Klüver-Barrera staining (double staining of Luxol Fast Blue staining and Nissl staining) and immunostaining (Iba1, CD3, NFP and MBP). Specimens were observed under an optical microscope, and then the presence or the absence of T cell and macrophage infiltration, and degeneration of myelin sheaths and axons were evaluated.

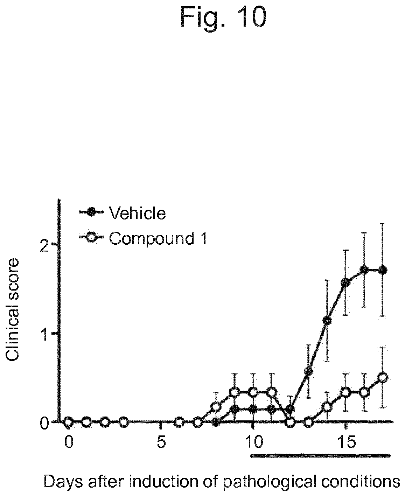

The results of evaluating the effect of compound 1 on clinical scores are shown in Figure 10. In Figure 10, the vertical axis indicates clinical scores, and the lower numerical value symptoms are improved (mean value ± standard error, n = 6-7 per group). Increases in clinical scores were suppressed in the compound 1 group, compared with the vehicle group.

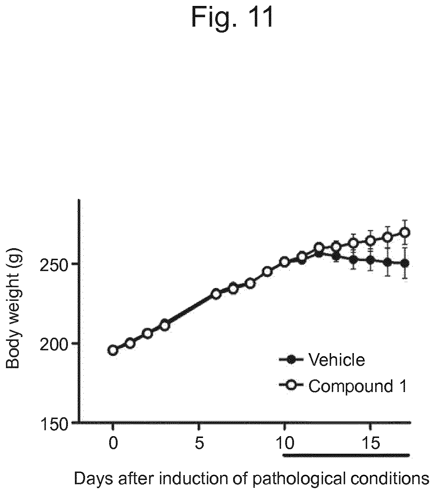

The results of evaluating the effect of compound 1 on body weight loss are shown in Figure 11. In Figure 11, the vertical axis indicates rat body weights (mean value ± standard error, n = 6 to 7 per group). Body weight loss was induced in the vehicle group, but no body weight loss was induced in the compound 1 group.

The results of histopathological evaluation for compound 1 are shown in Table 2. Table 2 shows the number of animals exhibiting histological changes in the sciatic nerve and tibial nerve (n = 3 per group). In the vehicle group, T cell and macrophage infiltration, and degeneration of myelin sheaths and axons were observed, however, almost no such alteration was observed in the compound 1 group.

**[Table 2]**

| | | **Vehicle group** | **Compound 1 administration group** |
|---|---|---|---|
| | | n = 3 | n = 3 |
| **Sciatic nerve** | | | |
| | **T cell infiltration** | 3/3 | 1/3 |
| | **Macrophage infiltration** | 3/3 | 1/3 |
| | **Myelin sheath degeneration** | 3/3 | 0/3 |
| | **Axonal degeneration** | 3/3 | 0/3 |

| **Tibial nerve** | | | |
|---|---|---|---|
| | **T cell infiltration** | 2/3 | 0/3 |
| | **Macrophage infiltration** | 2/3 | 0/3 |
| | **Myelin sheath degeneration** | 3/3 | 0/3 |
| | **Axonal degeneration** | 3/3 | 0/3 |

As described above, it was revealed that the cyclic amine derivative (I) or a pharmacologically acceptable salt thereof is effective against autoimmune peripheral neuropathies.

(Example 10) Effects of the cyclic amine derivative (I) or a pharmacologically acceptable salt thereof on mechanical allodynia in rat experimental autoimmune neuritis (EAN) model:
The suppressive effect of the cyclic amine derivative (I) or a pharmacologically acceptable salt thereof on mechanical allodynia in a rat EAN model was investigated.

EAN model rats were prepared in a manner similar to Example 9. Further, a pseudo-induction animal was prepared by administering physiological saline (Otsuka Pharmaceutical Factory, Inc.) instead of a peptide solution for administration. The day of administration of the peptide solution for administration or physiological saline was designated as day 0 after induction of pathological conditions.

On day 14 after induction of pathological conditions, compound 1 (5 or 10 mg/kg) or a solvent thereof (water for injection) was orally administered to rats. Groups were composed of 4 groups: a pseudo-induction and solvent administration group (sham group); a pathological condition-induced and solvent administration group (vehicle group); a pathological condition-induced and compound 1 (5 mg/kg) administration group (compound 1 (5 mg/kg) group); and a pathological condition-induced and compound 1 (10 mg/kg) administration group (compound 1 (10 mg/kg) administration group).

Beneficial effects on mechanical allodynia were evaluated in a manner similar to Example 5, and this evaluation was performed at 3 hours after administration of compound 1 on day 14 after induction of pathological conditions.

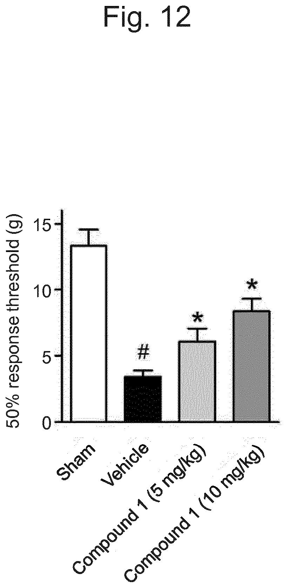

The results of evaluating the effects of compound 1 on mechanical allodynia are shown in Figure 12. In Figure 12, the vertical axis indicates 50% response thresholds in von Frey test, and the higher numerical value indicates that mechanical allodynia is improved (mean value ± standard error, n = 4 to 10 per group). The symbol "#" in Figure 12 indicates a statistically significant (#: p<0.05, Student's t-test) difference compared with the sham group and the symbols "*" in Figure 12 indicate statistically significant (*: p<0.025, Williams' multiple comparison, one sided) differences compared with the vehicle group.

A significant decrease in 50% response threshold was observed in the vehicle group compared with the sham group. Specifically, the development of mechanical allodynia in the EAN model was confirmed.

Significant increases in 50% response threshold were observed in the compound 1 (5 mg/kg) group and the compound 1 (10 mg/kg) group, compared with the vehicle group. Specifically, it was revealed that compound 1 suppresses mechanical allodynia in the EAN model.

These results revealed that the cyclic amine derivative (I) or a pharmacologically acceptable salt thereof is effective against mechanical allodynia in autoimmune peripheral neuropathies.

(Example 11) Effects of the cyclic amine derivative (I) or a pharmacologically acceptable salt thereof on decreased nerve conduction velocity in rat streptozotocin-induced diabetes model:
The suppressive effect of the cyclic amine derivative (I) or a pharmacologically acceptable salt thereof on mechanical allodynia in a rat streptozotocin-induced diabetes model was investigated.

Streptozotocin (50 mg/kg, Sigma-Aldrich) was administered intravenously to SD rats (6 weeks old, male; CHARLES RIVER LABORATORIES JAPAN, INC.) via the tail vein, thereby preparing the streptozotocin-induced diabetes model. Streptozotocin was dissolved in a citrate buffer to prepare a 25 mg/mL solution, and then administered. A no pathological condition-induced animal was prepared by administering no streptozotocin solution. The day of initial administration was designated as day 1 after induction of pathological conditions.

On days 14 to 28 after induction of pathological conditions, a solution containing compound 1 (3 or 10 mg/kg) or a solvent thereof (water for injection) was orally administered to rats twice a day. Groups were composed of 4 groups: a no pathological condition-induced and solvent administration group (normal group); a pathological condition-induced and solvent administration group (vehicle group); a pathological condition-induced and compound 1 (3 mg/kg) administration group (compound 1 (3 mg/kg) group); and a pathological condition-induced and compound 1 (10 mg/kg) administration group (compound 1 (10 mg/kg) group).

Nerve conduction velocity was measured within 3 days from the next day of the final administration. Two monopolar needle electrodes (A, B) were inserted into a femoral region for the electrodes to come into contact with the sciatic nerve. Another monopolar needle electrode (C) was inserted into the lower end portion of the gastrocnemius muscle (Achilles tendon), so as to set the lead-out electrode at the footpad. A-B stimulation and B-C stimulation were designated as distal stimulation and proximal stimulation, respectively, and then the transmission time of stimulation was analyzed on the basis of each lead-out waveform obtained from the electrode at the footpad. Nerve conduction velocity was calculated on the basis of a difference in transmission time between the distal stimulation and the proximal stimulation and the distance between the electrodes.

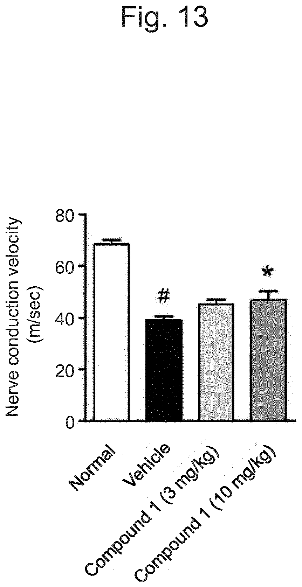

The results of evaluating the effects of compound 1 on decreased nerve conduction velocity are shown in Figure 13. In Figure 13, the vertical axis indicates nerve conduction velocity (mean value ± standard error; n = 5 to 6 per group). The symbol "#" in Figure 13 indicates a statistically significant (#: p<0.05, Student's t-test) difference compared with the normal group, and the symbol "*" in Figure 13 indicates a statistically significant (*: p<0.025, Williams' multiple comparison, one sided) difference compared with the vehicle group.

Significant increases in nerve conduction velocity as a result of repeated dosing of compound 1 were observed in the compound 1 (10 mg/kg) group, compared with the vehicle group. Specifically, it was revealed that compound 1 suppresses decreased nerve conduction velocity in the streptozotocin-induced diabetes model.

(Example 12) Effects of the cyclic amine derivative (I) or a pharmacologically acceptable salt thereof on mechanical allodynia in rat streptozotocin-induced diabetes model:
The suppressive effect of the cyclic amine derivative (I) or a pharmacologically acceptable salt thereof on mechanical allodynia in a rat streptozotocin-induced diabetes model was studied.

Streptozotocin (50 mg/kg, Sigma-Aldrich) was administered intravenously to SD rats (6 weeks old, male; CHARLES RIVER LABORATORIES JAPAN, INC.) via the tail vein, thereby preparing a streptozotocin-induced diabetes model. Streptozotocin was dissolved in physiological saline (Otsuka Pharmaceutical Factory, Inc.) to prepare a 25 mg/mL solution and then administered. Further, a pseudo-induction animal was also prepared by administering physiological saline instead of the streptozotocin solution. The initial day of administration was designated as day 0 after induction of pathological conditions.

On day 28 after induction of pathological conditions, a solution containing compound 1 (3 or 10 mg/kg) or a solvent thereof (water for injection) was orally administered to rats. Groups were composed of 4 groups: a pseudo-induction and solvent administration group (sham group); a pathological condition-induced and solvent administration group (vehicle group); a pathological condition-induced and compound 1 (10 mg/kg) administration group (compound 1 (10 mg/kg) group); and a pathological condition-induced and compound 1 (30 mg/kg) administration group (compound 1 (30 mg/kg) group).

Beneficial effects on mechanical allodynia were evaluated in a manner similar to Example 5, and this evaluation was performed at 3 hours after administration of compound 1 on day 28 after induction of pathological conditions.

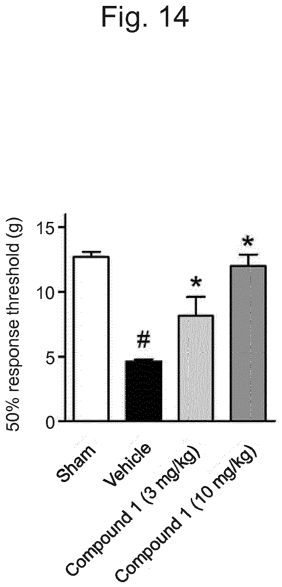

The results of evaluating the effects of compound 1 on mechanical allodynia are shown in Figure 14. In Figure 14, the vertical axis indicates 50% response thresholds in von Frey test, and the higher numerical valueindicates that mechanical allodynia is improved (mean value ± standard error; n = 8 per group). The symbols "*" in Figure 14 indicate statistically significant (*: p<0.025, Shirley-Williams' multiple comparison, one sided) differences compared with the vehicle group.

On day 28 after induction of pathological conditions (3 hours after administration of compound 1), significant increases in 50% response threshold were observed in the compound 1 (10 mg/kg) group and the compound 1 (30 mg/kg) group, compared with the vehicle group. Specifically, it was revealed that compound 1 suppresses mechanical allodynia in the streptozotocin-induced diabetes model.

As described above, it was revealed that the cyclic amine derivative (I) or a pharmacologically acceptable salt thereof is effective against metabolic peripheral neuropathies, and particularly diabetic peripheral neuropathy.

### INDUSTRIAL APPLICABILITY

The cyclic amine derivative or a pharmacologically acceptable salt thereof of the present invention has effects of protecting and repairing peripheral neurons and effects of accelerating myelination, and significantly suppresses the symptoms and the like of various peripheral neuropathies, and thus can be used as a therapeutic or prophylactic agent for peripheral neuropathies.

All publications, patents, and patent applications cited herein are incorporated herein by reference in their entirety.

## Claims

1. A therapeutic or prophylactic agent for peripheral neuropathies, comprising as an active ingredient a cyclic amine derivative represented by general formula (I) or a pharmacologically acceptable salt thereof 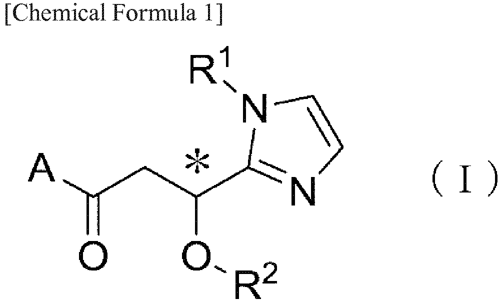 [wherein,
carbon marked with * is asymmetric carbon; and
A represents a group represented by general formulae (IIa), (IIb) or (IIc): 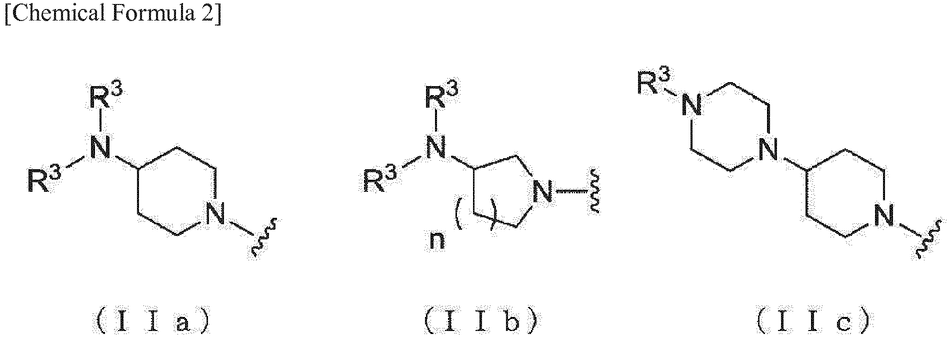
wherein R¹ represents a methyl group or an ethyl group optionally substituted with a halogen atom,
R² represents a hydrogen atom or an alkylcarbonyl group having 2 to 5 carbon atoms,
each R³ independently represents a methyl group or an ethyl group, and
n represents 1 or 2.].

2. The therapeutic or prophylactic agent according to claim 1, wherein A is the group represented by general formula (IIa).

3. The therapeutic or prophylactic agent according to claim 1, wherein A is the group represented by general formulae (IIb) or (IIc).

4. The therapeutic or prophylactic agent according to claim 1, wherein A is the group represented by general formula (IIa) and the stereochemical configuration of the asymmetric carbon marked with * is S.

5. The therapeutic or prophylactic agent according to any one of claims 1 to 4, wherein R¹ represents a methyl group or an ethyl group optionally substituted with a fluorine atom.

6. The therapeutic or prophylactic agent according to any one of claims 1 to 4, wherein R¹ is a methyl group, an ethyl group, a difluoromethyl group or a 2,2,2-trifluoroethyl group.

7. A therapeutic or prophylactic agent for drug-induced peripheral neuropathies, comprising as an active ingredient a cyclic amine derivative represented by general formula (I) or a pharmacologically acceptable salt thereof 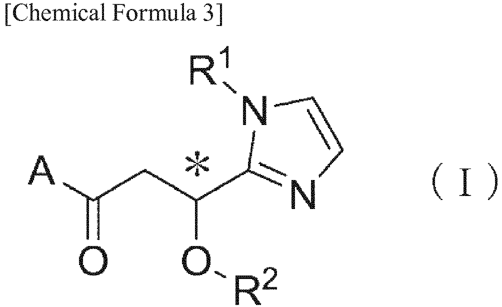 [wherein,
carbon marked with * is asymmetric carbon; and
A represents a group represented by general formulae (IIa), (IIb) or (IIc): 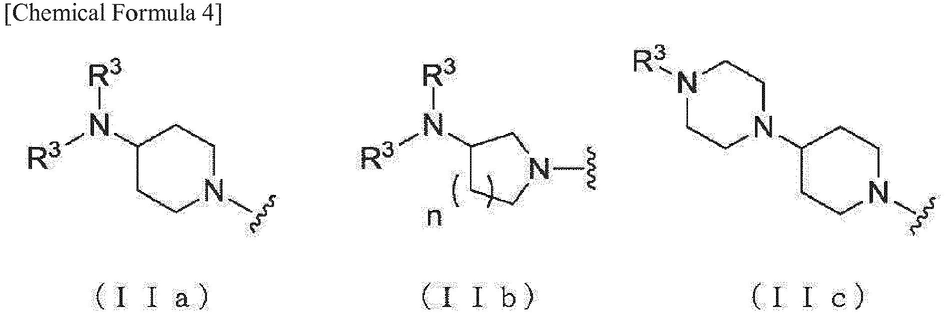
wherein R¹ represents a methyl group or an ethyl group optionally substituted with a halogen atom,
R² represents a hydrogen atom or an alkylcarbonyl group having 2 to 5 carbon atoms,
each R³ independently represents a methyl group or an ethyl group, and
n represents 1 or 2.].

8. The therapeutic or prophylactic agent according to claim 7, wherein A is the group represented by general formula (IIa).

9. The therapeutic or prophylactic agent according to claim 7, wherein A is the group represented by general formulae (IIb) or (IIc).

10. The therapeutic or prophylactic agent according to claim 7, wherein A is the group represented by general formula (IIa), and the stereochemical configuration of the asymmetric carbon marked with * is S.

11. The therapeutic or prophylactic agent according to any one of claims 7 to 10, wherein R¹ represents a methyl group or an ethyl group optionally substituted with a fluorine atom.

12. The therapeutic or prophylactic agent according to any one of claims 7 to 10, wherein R¹ is a methyl group, an ethyl group, a difluoromethyl group or a 2,2,2-trifluoroethyl group.

13. The therapeutic or prophylactic agent according to any one of claims 7 to 12, wherein the drug-induced peripheral neuropathies are at least one type selected from anticancer agent-induced peripheral neuropathy, antiviral agent-induced peripheral neuropathy, antimicrobial agent-induced peripheral neuropathy, antitubercular agent-induced peripheral neuropathy, antiarrhythmic agent-induced peripheral neuropathy, lipid-lowering drug-induced peripheral neuropathy, immunosuppressive drug-induced peripheral neuropathy, gout therapeutic agent-induced peripheral neuropathy and peripheral neuropathies induced by other drugs.

14. A therapeutic or prophylactic agent for autoimmune peripheral neuropathies, comprising as an active ingredient a cyclic amine derivative represented by general formula (I) or a pharmacologically acceptable salt thereof 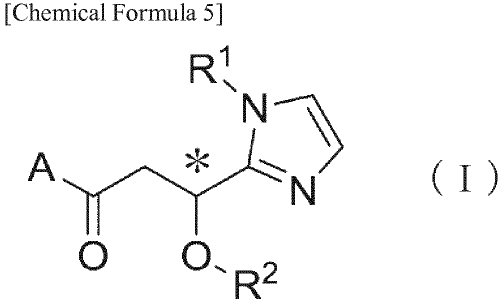 [wherein,
carbon marked with * is asymmetric carbon; and
A represents a group represented by general formulae (IIa), (IIb) or (IIc): 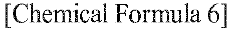 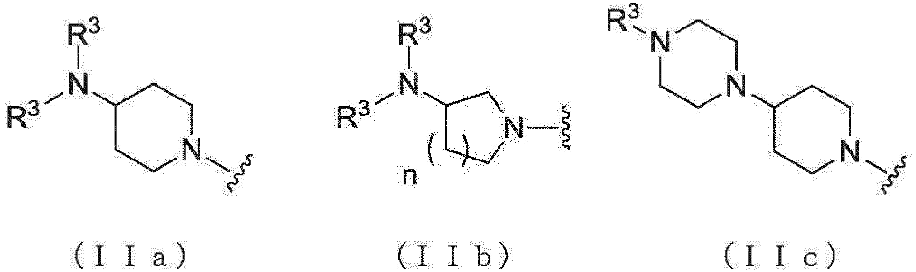
wherein R¹ represents a methyl group or an ethyl group optionally substituted with a halogen atom,
R² represents a hydrogen atom or an alkylcarbonyl group having 2 to 5 carbon atoms,
each R³ independently represents a methyl group or an ethyl group, and
n represents 1 or 2.].

15. The therapeutic or prophylactic agent according to claim 14, wherein A is the group represented by general formula (IIa).

16. The therapeutic or prophylactic agent according to claim 14, wherein A is the group represented by general formulae (IIb) or (IIc).

17. The therapeutic or prophylactic agent according to claim 14, wherein A is the group represented by general formula (IIa), and the stereochemical configuration of the asymmetric carbon marked with * is S.

18. The therapeutic or prophylactic agent according to any one of claims 14 to 17, wherein R¹ represents a methyl group or an ethyl group optionally substituted with a fluorine atom.

19. The therapeutic or prophylactic agent according to any one of claims 14 to 17, wherein R¹ is a methyl group, an ethyl group, a difluoromethyl group or a 2,2,2-trifluoroethyl group.

20. The therapeutic or prophylactic agent according to any one of claims 14 to 19, wherein autoimmune peripheral neuropathies are at least one type selected from Guillain-Barré syndrome, chronic inflammatory demyelinating polyradiculoneuropathy, multifocal motor neuropathy and paraproteinemic neuropathy.

21. A therapeutic or prophylactic agent for metabolic peripheral neuropathies, comprising as an active ingredient a cyclic amine derivative represented by general formula (I) or a pharmacologically acceptable salt thereof 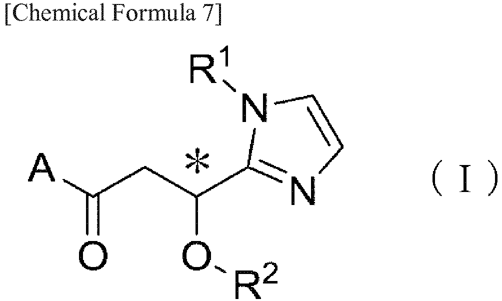 [wherein,
carbon marked with * is asymmetric carbon; and
A represents a group represented by general formulae (IIa), (IIb) or (IIc): 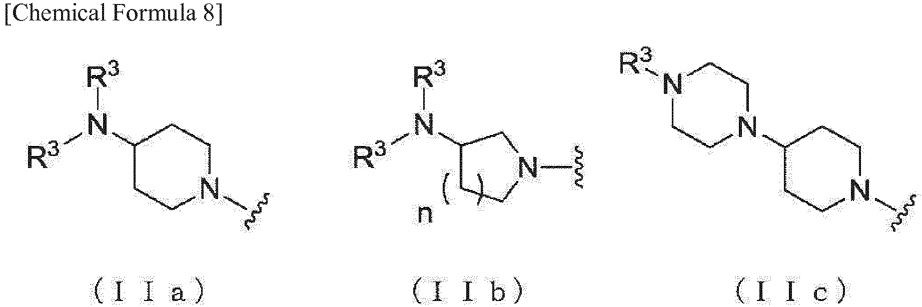
wherein R¹ represents a methyl group or an ethyl group optionally substituted with a halogen atom,
R² represents a hydrogen atom or an alkylcarbonyl group having 2 to 5 carbon atoms,
each R³ independently represents a methyl group or an ethyl group, and
n represents 1 or 2.].

22. The therapeutic or prophylactic agent according to claim 21, wherein A is the group represented by general formula (IIa).

23. The therapeutic or prophylactic agent according to claim 21, wherein A is the group represented by general formulae (IIb) or (IIc).

24. The therapeutic or prophylactic agent according to claim 21, wherein A is the group represented by general formula (IIa), and the stereochemical configuration of the asymmetric carbon marked with * is S.

25. The therapeutic or prophylactic agent according to any one of claims 21 to 24, wherein R¹ represents a methyl group or an ethyl group optionally substituted with a fluorine atom.

26. The therapeutic or prophylactic agent according to any one of claims 21 to 24, wherein R¹ is a methyl group, an ethyl group, a difluoromethyl group or a 2,2,2-trifluoroethyl group.

27. The therapeutic or prophylactic agent according to any one of claims 21 to 26, wherein metabolic peripheral neuropathies are at least one type selected from diabetic peripheral neuropathy, uremic peripheral neuropathy, collagen-peripheral neuropathy and vitamin deficiency peripheral neuropathy.

28. A therapeutic or prophylactic agent for hereditary peripheral neuropathies, comprising as an active ingredient a cyclic amine derivative represented by general formula (I) or a pharmacologically acceptable salt thereof 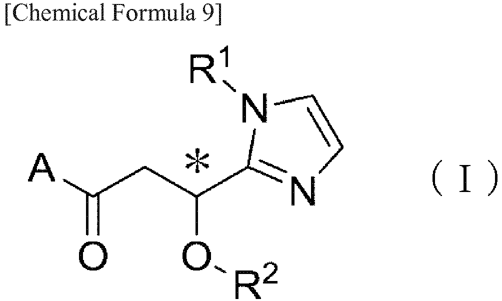 [wherein,
carbon marked with * is asymmetric carbon; and
A represents a group represented by general formulae (IIa), (IIb) or (IIc): 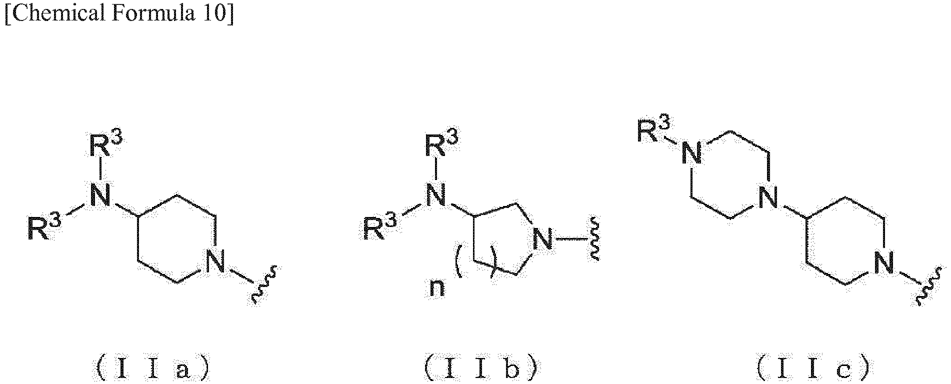
wherein R¹ represents a methyl group or an ethyl group optionally substituted with a halogen atoms,
R² represents a hydrogen atom or an alkylcarbonyl group having 2 to 5 carbon atoms,
each R³ independently represents a methyl group or an ethyl group, and
n represents 1 or 2.].

29. The therapeutic or prophylactic agent according to claim 28, wherein A is the group represented by general formula (IIa).

30. The therapeutic or prophylactic agent according to claim 28, wherein A is the group represented by general formulae (IIb) or (IIc).

31. The therapeutic or prophylactic agent according to claim 28, wherein A is the group represented by general formula (IIa), and the stereochemical configuration of the asymmetric carbon marked with * is S.

32. The therapeutic or prophylactic agent according to any one of claims 28 to 31, wherein R¹ represents a methyl group or an ethyl group optionally substituted with a fluorine atom.

33. The therapeutic or prophylactic agent according to any one of claims 28 to 31, wherein R¹ is a methyl group, an ethyl group, a difluoromethyl group or a 2,2,2-trifluoroethyl group.

34. The therapeutic or prophylactic agent according to any one of claims 28 to 33, wherein the hereditary peripheral neuropathies are at least one type selected from Charcot-Marie-Tooth disease, familial amyloid polyneuropathy, hereditary neuropathy to pressure palsies (HNPP) and hereditary neuralgic amyotrophy.
